# EUROPEAN PATENT APPLICATION

(11) **EP 4 227 293 A2**
(43) Date of publication of application: **16.08.2023**
(21) Application number: 22204152.7
(22) Date of filing: 14.05.2019
(51) Int. Cl.: C07C 271/22, A61K 31/325, A61P 25/00

(54) **CRYSTALLINE FORMS OF 1-(ACYLOXY)-ALKYL CARBAMATE DRUG CONJUGATES OF NAPROXEN AND PREGABALIN**

(30) Priority: 14.05.2018 WO PCT/CN2018/086755
(62) Divisional of application: 19803008.2
(71) Applicant: Xgene Pharmaceutical Inc, Grand Cayman KY1-1112 (KY)
(72) Inventor: XU, Feng, Palo Alto, California, 94303 (US); LI, Weidong, Chengdu, Sichuan, 610031 (CN); CHEN, Li, Tianjin, 300071 (CN); AI, Xinmiao, Beijing, 100176 (CN)
(74) Representative: Mewburn Ellis LLP

(57) **Abstract**

Crystalline forms of the drug conjugate (*S*)-3-((((*R*)-1-((*S*)-2-(6-methoxynaphthalen-2-yl)propanoyloxy)ethoxy)carbonyl-amino)methyl)-5-methylhexanoic acid and their pharmaceutical compositions are described herein. Also described are methods of using the crystalline forms for treatment of diseases and conditions.

## Description

### CROSS-REFERENCE

This patent application claims the benefit of PCT Application No. PCT/CN2018/086755, filed May 14, 2018; which is incorporated herein by reference in its entirety.

### BACKGROUND

(*S*)-3-(((*R*)-1-((*S*)-2-(6-methoxynaphthalen-2-yl)propanoyloxy)ethoxy)carbonyl-amino)methyl)-5-methylhexanoic acid is a drug conjugate of pregabalin and naproxen.

Amorphous and crystalline forms of a drug have different physical/chemical properties and/or bioavailabilities. A key characteristic of any crystalline substance is the polymorphic behavior. Polymorphs are crystals of the same molecule which have different physical properties because the crystal lattice contains a different arrangement of the molecules. The different physical properties exhibited by polymorphs affect the pharmaceutical parameters such as storage, stability, compressibility, density and dissolution rates. These properties are important in formulation, manufacturing and bioavailability of a drug product. The solubility differences between polymorphs may, in extreme situations, result in transition to crystalline forms that lack potency or even unwanted toxicity. In addition, the physical properties of the crystalline form may be important in pharmaceutical processing.

There still remains a need for polymorphs of (*S*)-3-((((*R*)-1-((*S*)-2-(6-methoxynaphthalen-2-yl)propanoyloxy)ethoxy)carbonyl-amino)methyl)-5-methylhexanoic acid. This invention addresses this need and provides related advantages as well.

### SUMMARY

In one aspect, provided herein is a composition comprising a crystalline form of ((*S*)-3-((((*R*)-1-((S)-2-(6-methoxynaphthalen-2-yl)propanoyloxy)ethoxy)carbonylamino)methyl)-5-methylhexanoic acid) ("the compound of Formula I") which is a conjugate of pregabalin and naproxen. In animal studies, the compound of Formula I is absorbed efficiently after oral dosing and rapidly converted to naproxen and pregabalin upon absorption. Preclinical toxicology and safety pharmacology studies and phase I clinical studies in healthy volunteers demonstrate that the compound of Formula I does not cause any pharmacological effects beyond those expected from exposure to naproxen and pregabalin.

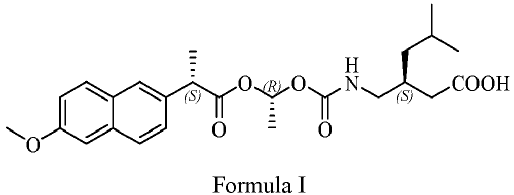

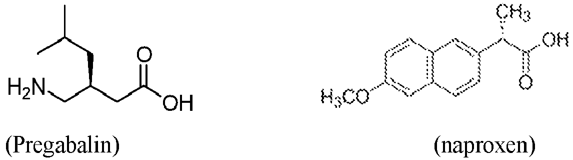

In one aspect, the invention provides a composition comprising a crystalline form of the compound of Formula I. In some embodiments, the crystalline form is characterized by having X-ray powder diffraction (XRPD) peaks at about 8.6, 15.5, 16.4, 17.9, and 20.6 degrees 2Θ. In some embodiments, the crystalline form is characterized by having at least one additional XRPD peak at about 6.4, 17.2, 17.5, 20.4, 21.4, 22.4, 23.7, 23.9, or 27.6 degrees 2Θ. In some embodiments, the crystalline form is characterized by having at least one additional XRPD peak at about 9.3, 9.8, 19.9, 21.7, 22.9, 24.3, 25.0, 25.6, 26.4, 26.9, 29.7, or 31.3 degrees 2Θ.

In some embodiments, the crystalline form is characterized by having XRPD peaks at about 6.4, 8.6, 15.5, 16.4, 17.2, 17.5, 17.9, 20.4, 20.6, 21.4, 22.4, 23.7, 23.9, and 27.6 degrees 2Θ. In some embodiments, the crystalline form is characterized by having at least one additional XRPD peak at about 9.3, 9.8, 19.9, 21.7, 22.9, 24.3, 25.0, 25.6, 26.4, 26.9, 29.7, or 31.3 degrees 2Θ.

In some embodiments, the crystalline form is characterized by having XRPD peaks at about 6.4, 8.6, 9.3, 9.8, 15.5, 16.4, 17.2, 17.5, 17.9, 19.9, 20.4, 20.6, 21.4, 21.7, 22.4, 22.9, 23.7, 23.9, 24.3, 25.0, 25.6, 26.4, 26.9, 27.6, 29.7, and 31.3 degrees 2Θ.

In some embodiments, the crystalline form has a chemical purity of greater than about 90%. In some embodiments, the chemical purity of the crystalline from is determined by HPLC analysis. In some embodiments, the crystalline from has an enantiomeric purity of greater than about 90%. In some embodiments, the crystalline from has a diastereomeric purity of greater than about 90%.

In some embodiments, the crystalline form has a melting point in the range of from about 100° C to about 140° C. In some embodiments, the crystalline form has a melting point in the range of from about 110 °C to about 130 °C. In some embodiments, the crystalline form has a melting point in the range of from about from about 118 °C to about 121 °C. In some embodiments, the crystalline form has a melting point in the range of from about from about 119 °C to about 121 °C.

In some embodiments, the composition comprises at least two crystalline forms of the compound of Formula I.

In another aspect, the disclosure provides a pharmaceutical composition comprising the crystalline forms disclosed herein. In some embodiments, the pharmaceutical composition is in a solid dosage form. In some embodiments, the pharmaceutical composition is a capsule. In some embodiments, the pharmaceutical composition is a liquid. In some embodiments, the pharmaceutical composition is a suspension. In some embodiments, the pharmaceutical composition is an aqueous suspension. In some embodiments, the pharmaceutical composition comprises a solution of a crystalline form in water. In some embodiments, the pharmaceutical composition comprises a solution of a crystalline form in a saline solution, an aqueous dextrose solution, a glycerol solution, or a combination thereof. In some embodiments, the pharmaceutical composition is for intravenous administration. In some embodiments, the pharmaceutical composition is for oral administration.

In some embodiments, the pharmaceutical composition provided herein further comprises one or more excipients selected from the group consisting of wetting agents, emulsifying agents, buffering agents, stabilizing agents, thickening agents, lubricating agents, and coloring agents. In some embodiments, the pharmaceutical composition comprises at least two crystalline forms of the compound of Formula I.

In another aspect, the disclosure provides a kit for preventing or treating a disease in a subject, the kit comprising the composition or the pharmaceutical composition of the disclosure and instructions for using the kit. In some embodiments, the subject is an animal. In some embodiments, the subject is a human. In some embodiments, the disease is a pain or an inflammatory disease. In some embodiments, the pain is a neuropathic pain or a musculoskeletal pain. In some embodiments, the inflammatory disease is arthritis. In some embodiments, the kit further comprises at least one additional therapeutic agent. In some embodiments, the at least one additional therapeutic agent is an agent for treatment of a pain or a neurological disease. In some embodiments, the composition or the pharmaceutical composition of the disclosure and the at least one additional therapeutic agent act additively. In some embodiments, the composition or the pharmaceutical composition of the disclosure and the at least one additional therapeutic agent act synergistically.

In another aspect, the disclosure provides a method of treating or preventing a disease in a subject in need thereof, wherein the method comprises administering to the subject an effective amount of the composition or the pharmaceutical composition of the disclosure. In some embodiments, the subject is a human. In some embodiments, the disease is a pain or an inflammatory disease. In some embodiments, the pain is a neuropathic pain or a musculoskeletal pain. In some embodiments, the inflammatory disease is arthritis.

In some embodiments, the method of treating disclosed herein further comprises administering at least one additional therapeutic agent to the subject. In some embodiments, the at least one additional therapeutic agent is an agent for treatment of a pain or a neurological disease. In some embodiments, the composition or the pharmaceutical composition of the disclosure and the at least one additional therapeutic agent act additively. In some embodiments, the composition or the pharmaceutical composition of the disclosure and the at least one additional therapeutic agent act synergistically. In some embodiments, the composition or the pharmaceutical composition of the disclosure and the at least one additional therapeutic agent are administered concurrently.

In another aspect, the disclosure provides a method of making a crystalline form of a compound of Formula I, herein the method comprises: (i) dissolving a composition comprising the compound of Formula I in a solvent to obtain a solution of the compound of Formula I; and (ii) isolating the crystalline form from the solution of the compound of Formula I. In some embodiments, the crystalline form obtained is characterized by having XRPD peaks at about 8.6, 15.5, 16.4, 17.9, and 20.6 degrees 2Θ. In some embodiments, the crystalline form obtained is characterized by having at least one additional XRPD peak at about 6.4, 17.2, 17.5, 20.4, 21.4, 22.4, 23.7, 23.9, or 27.6 degrees 2Θ. In some embodiments, the crystalline form obtained is characterized by having XRPD peaks at about 6.4, 8.6, 15.5, 16.4, 17.2, 17.5, 17.9, 20.4, 20.6, 21.4, 22.4, 23.7, 23.9, and 27.6 degrees 2Θ. In some embodiments, the crystalline form obtained is characterized by having at least one additional XRPD peak at about 9.3, 9.8, 19.9, 21.7, 22.9, 24.3, 25.0, 25.6, 26.4, 26.9, 29.7 or 31.3 degrees 2Θ.

In some embodiments, the step of dissolving comprises heating a mixture of the composition comprising the compound of Formula I and the solvent to a temperature above an ambient temperature. In some embodiments, the step of isolating the crystalline form from the solution of the compound of Formula I comprises cooling the solution of the compound of Formula I to a temperature below an ambient temperature. In some embodiments, the step of isolating the crystalline form from the solution of the compound of Formula I comprises cooling the solution of the compound of Formula I to a temperature of between about 0 °C and 25 °C.

In some embodiments, the method of making the crystalline form of the compound of Formula I provided herein further comprises adding an additional solvent to the solution of the compound of Formula I.

In some embodiments, the solvent comprises a polar protic solvent. For example, in some embodiments, the solvent comprises isopropanol. In some embodiments, the step of dissolving comprises heating a mixture of the composition comprising the compound of Formula I and the solvent to a temperature of about 40 °C to about reflux temperature. In some embodiments, the additional solvent comprises an alkane. In some embodiments, the additional solvent comprises heptane. In some embodiments, the solvent and the additional solvent are used in a volume ratio of about 1:2 to about 1:3 (v/v).

In some embodiments, the solvent comprises heptane, ethyl acetate, or a mixture thereof. In some embodiments, the solvent comprises heptane and ethyl acetate in ratio of about 10:1 by volume. In some embodiments, the step of dissolving comprises heating a mixture of the composition comprising the compound of Formula I and the solvent to a temperature of about 50 °C to about reflux temperature. In some embodiments the step of dissolving comprises heating a mixture of the composition comprising the compound of Formula I and the solvent to a temperature of about 70 °C.

In some embodiments, the solvent comprises methylcyclohexane, methyl t-butyl ether, or a mixture thereof. In some embodiments, the solvent comprises methylcyclohexane and methyl t-butyl ether in ratio of about 10:1 by volume. In some embodiments, the step of dissolving comprises heating a mixture of the composition comprising the compound of Formula I and the solvent to a temperature of about 20 °C to about 40 °C.

In various embodiments, the method of making the crystalline form of the compound of Formula I provided herein further comprises introducing a seed crystalline form into the solution of the compound of Formula I.

Disclosed herein is a crystalline form of a compound of Formula I: 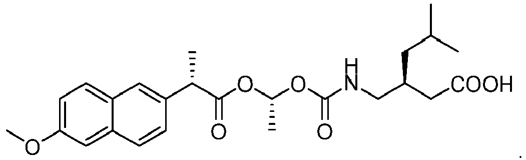 In some embodiments of the crystalline form, the crystalline form is characterized by having X-ray powder diffraction (XRPD) peaks at about 8.6, 15.5, 16.4, 17.9, and 20.6 degrees 2Θ. In some embodiments of the crystalline form, the crystalline form is characterized by having at least one additional XRPD peak at about 6.4, 17.2, 17.5, 20.4, 21.4, 22.4, 23.7, 23.9, or 27.6 degrees 20 In some embodiments of the crystalline form, the crystalline form is characterized by having XRPD peaks at about 6.4, 8.6, 15.5, 16.4, 17.2, 17.5, 17.9, 20.4, 20.6, 21.4, 22.4, 23.7, 23.9, and 27.6 degrees 2Θ. In some embodiments of the crystalline form, the crystalline form is characterized by having at least one additional XRPD peak at about 9.3, 9.8, 19.9, 21.7, 22.9, 24.3, 25.0, 25.6, 26.4, 26.9, 29.7 or 31.3 degrees 2Θ. In some embodiments of the crystalline form, the crystalline form is characterized by having XRPD peaks at about 6.4, 8.6, 9.3, 9.8, 15.5, 16.4, 17.2, 17.5, 17.9, 19.9, 20.4, 20.6, 21.4, 21.7, 22.4, 22.9, 23.7, 23.9, 24.3, 25.0, 25.6, 26.4, 26.9, 27.6, 29.7 and 31.3 degrees 2Θ. In some embodiments of the crystalline form, the crystalline form has a chemical purity of greater than about 90%. In some embodiments of the crystalline form, the chemical purity of the crystalline from is determined by HPLC analysis. In some embodiments of the crystalline form, the crystalline from has an enantiomeric purity of greater than about 90%. In some embodiments of the crystalline form, the crystalline from has a diastereomeric purity of greater than about 90%. In some embodiments of the crystalline form, the crystalline form has a melting point in the range of from about 100° C to about 140° C. In some embodiments of the crystalline form, the crystalline form has a melting point in the range of from about 110 °C to about 130 °C. In some embodiments of the crystalline form, the crystalline form has a melting point in the range of from about from about 118 °C to about 121 °C. In some embodiments of the crystalline form, the crystalline form has a melting point in the range of from about from about 119 °C to about 121 °C. In some embodiments of the crystalline form, the composition comprises at least two crystalline forms of the compound of Formula I.

Also disclosed herein is a pharmaceutical composition comprising the crystalline form disclosed herein. In some embodiments of the pharmaceutical composition, the pharmaceutical composition is in a solid dosage form. In some embodiments of the pharmaceutical composition, the pharmaceutical composition is a capsule. In some embodiments of the pharmaceutical composition, the pharmaceutical composition is a suspension. In some embodiments of the pharmaceutical composition, the pharmaceutical composition is an aqueous suspension. In some embodiments of the pharmaceutical composition, the pharmaceutical composition is a liquid. In some embodiments of the pharmaceutical composition, the pharmaceutical composition comprises a solution of the crystalline form in water. In some embodiments of the pharmaceutical composition, the pharmaceutical composition comprises a solution of the crystalline form in a saline solution, an aqueous dextrose solution, a glycerol solution, or a combination thereof. In some embodiments of the pharmaceutical composition, the pharmaceutical composition is for intravenous administration. In some embodiments of the pharmaceutical composition, the pharmaceutical composition is for oral administration. In some embodiments of the pharmaceutical composition, the pharmaceutical composition further comprises one or more excipients selected from the group consisting of wetting agents, emulsifying agents, buffering agents, stabilizing agents, thickening agents, lubricating agents, and coloring agents. In some embodiments of the pharmaceutical composition, the pharmaceutical composition comprises at least two crystalline forms of the compound of Formula I.

Also disclosed herein is a kit for preventing or treating a disease in a subject, the kit comprising the crystalline form disclosed herein, or the pharmaceutical composition disclosed herein, and instructions for using the kit. In some embodiments of the kit, the subject is an animal. In some embodiments of the kit, the subject is a human. In some embodiments of the kit, the disease is a pain or an inflammatory disease. In some embodiments of the kit, the pain is a neuropathic pain or a musculoskeletal pain. In some embodiments of the kit, the inflammatory disease is arthritis. In some embodiments of the kit, the kit further comprises at least one additional therapeutic agent. In some embodiments of the kit, the at least one additional therapeutic agent is an agent for treatment of a pain or a neurological disease. In some embodiments of the kit, the crystalline form or the pharmaceutical composition and the at least one additional therapeutic agent act additively. In some embodiments of the kit, the crystalline form or the pharmaceutical composition and the at least one additional therapeutic agent act synergistically.

Also disclosed herein is a method of treating or preventing a disease in a subject in need thereof, wherein the method comprises administering to the subject the pharmaceutical composition disclosed herein. In some embodiments of the method of treating or preventing a disease, the subject is a human. In some embodiments of the method of treating or preventing a disease, the disease is a pain or an inflammatory disease. In some embodiments of the method of treating or preventing a disease, the pain is a neuropathic pain or a musculoskeletal pain. In some embodiments of the method of treating or preventing a disease, the inflammatory disease is arthritis. In some embodiments of the method of treating or preventing a disease, the method further comprises administering at least one additional therapeutic agent to the subject. In some embodiments of the method of treating or preventing a disease, the at least one additional therapeutic agent is an agent for treatment of a pain or a neurological disease. In some embodiments of the method of treating or preventing a disease, the pharmaceutical composition and the at least one additional therapeutic agent act additively. In some embodiments of the method of treating or preventing a disease, the pharmaceutical composition and the at least one additional therapeutic agent act synergistically. In some embodiments of the method of treating or preventing a disease, the pharmaceutical composition and the at least one additional therapeutic agent are administered concurrently.

Also disclosed herein is a method of making a crystalline form of a compound of Formula I: 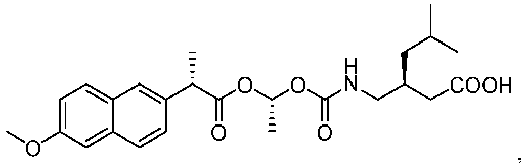 wherein the method comprises:
(i) dissolving a compound of Formula I in a solvent to obtain a solution of the compound of Formula I; and
(ii) isolating the crystalline form from the solution of the compound of Formula I.

In some embodiments of the method, the crystalline form is characterized by having XRPD peaks at about 8.6, 15.5, 16.4, 17.9, and 20.6 degrees 2Θ. In some embodiments of the method, the crystalline form is characterized by having at least one additional XRPD peak at about 6.4, 17.2, 17.5, 20.4, 21.4, 22.4, 23.7, 23.9, or 27.6 degrees 2Θ. In some embodiments of the method, the crystalline form is characterized by having XRPD peaks at about 6.4, 8.6, 15.5, 16.4, 17.2, 17.5, 17.9, 20.4, 20.6, 21.4, 22.4, 23.7, 23.9, and 27.6 degrees 2Θ. In some embodiments of the method, the crystalline form is characterized by having at least one additional XRPD peak at about 9.3, 9.8, 19.9, 21.7, 22.9, 24.3, 25.0, 25.6, 26.4, 26.9, 29.7 or 31.3 degrees 2Θ. In some embodiments of the method, the step of dissolving comprises heating the compound of Formula I and the solvent to a temperature above an ambient temperature. In some embodiments of the method, the step of isolating the crystalline form from the solution of the compound of Formula I comprises cooling the solution of the compound of Formula I to a temperature below an ambient temperature. In some embodiments of the method, the step of isolating the crystalline form from the solution of the compound of Formula I comprises cooling the solution of the compound of Formula I to a temperature of between about 0 °C and 25 °C. In some embodiments of the method, the method further comprises adding an additional solvent to the solution of the compound of Formula I. In some embodiments of the method, the solvent comprises a polar protic solvent. In some embodiments of the method, the solvent comprises isopropanol. In some embodiments of the method, the step of dissolving comprises heating the compound of Formula I and the solvent to a temperature of about 40 °C to about reflux temperature. In some embodiments of the method, the additional solvent comprises an alkane. In some embodiments of the method, the additional solvent comprises heptane. In some embodiments of the method, the solvent and the additional solvent are used in a volume ratio of about 1:2 to about 1:3 (v/v). In some embodiments of the method, the solvent comprises heptane, ethyl acetate, or a mixture thereof. In some embodiments of the method, the solvent comprises heptane and ethyl acetate in ratio of about 10:1 by volume. In some embodiments of the method, the step of dissolving comprises heating the compound of Formula I and the solvent to a temperature of about 50 °C to about reflux temperature. In some embodiments of the method, the step of dissolving comprises heating the compound of Formula I and the solvent to a temperature of about 70 °C. In some embodiments of the method, the solvent comprises methylcyclohexane, methyl t-butyl ether, or a mixture thereof. In some embodiments of the method, the solvent comprises methylcyclohexane and methyl t-butyl ether in ratio of about 10:1 by volume. In some embodiments of the method, the step of dissolving comprises heating the compound of Formula I and the solvent to a temperature of about 20 °C to about 40 °C. In some embodiments of the method, the method further comprises introducing a seed crystalline form into the solution of the compound of Formula I.

### BRIEF DESCRIPTION OF THE FIGURES

**FIG. 1** shows an X-ray powder diffractogram of the crystalline form of the compound of Formula I.
**FIG. 2** shows a FT-IR spectrum of the crystalline form of the compound of Formula I.
**FIG.3** shows a DSC & TGA overlay spectrum of the crystalline form of the compound of Formula I.
**FIG. 4** shows an X-ray structure of the compound of Formula I.

### DETAILED DESCRIPTION

### Definitions

Unless defined otherwise, all technical and scientific terms used herein have the same meaning as is commonly understood by one of skill in the art to which this invention belongs.

As used in the specification and claims, the singular form "a", "an" and "the" includes plural references unless the context clearly dictates otherwise.

"Pharmaceutically acceptable vehicle" refers to a diluent, adjuvant, excipient or carrier with which the compound of Formula I is administered.

"Patient" refers to an animal, such as a mammal, for example a human. The methods described herein can be useful in both human therapeutics and veterinary applications. In some embodiments, the patient is a mammal, and in some embodiments, the patient is human. The terms "patient" and "subject" are used interchangeably herein.

"Preventing" or "prevention" refers to a reduction in risk of acquiring a disease or disorder (i.e., causing at least one of the clinical symptoms of the disease not to develop in a patient that may be exposed to or predisposed to the disease but does not yet experience or display symptoms of the disease).

"Drug conjugate" refers to the biologically active drugs linked by chemical linkers with labile chemical bonds. Typically, the linker will be attached to the drugs *via* bond(s) that are cleaved by enzymatic or non-enzymatic means *in vivo.*

"Treating" or "treatment" of any disease or disorder refers, in one embodiment, to ameliorating the disease or disorder (i.e., arresting or reducing the development of the disease or at least one of the clinical symptoms thereof). In another embodiment "treating" or "treatment" refers to ameliorating at least one physical parameter, which may not be discernible by the patient. In yet another embodiment, "treating" or "treatment" refers to inhibiting the disease or disorder, either physically, (e. g., stabilization of a discernible symptom), physiologically, (e.g., stabilization of a physical parameter), or both. In yet another embodiment, "treating" or "treatment" refers to delaying the onset of the disease or disorder.

"Therapeutically effective amount" means the amount of a compound that, when administered to a patient for treating a disease, is sufficient to effect such treatment for the disease. The "therapeutically effective amount" will vary depending on the compound, the disease and its severity and the age, weight, etc., of the patient to be treated.

"Solvate" refers to a compound (e.g., a compound as described herein or a pharmaceutically acceptable salt thereof) in physical association with one or more molecules of a pharmaceutically acceptable solvent.

"Crystalline form," and "polymorph," may be used interchangeably herein, and are meant to include all crystalline and amorphous forms of the compound, including, for example, polymorphs, pseudopolymorphs, solvates, hydrates, unsolvated polymorphs (including anhydrates), conformational polymorphs, and amorphous forms, as well as mixtures thereof, unless a particular crystalline or amorphous form is referred to. Compounds of the present invention include crystalline and amorphous forms of those compounds, including, for example, polymorphs, pseudopolymorphs, solvates, hydrates, unsolvated polymorphs (including anhydrates), conformational polymorphs, and amorphous forms of the compounds, as well as mixtures thereof.

The term "co-administration," "administered in combination with," and their grammatical equivalents, as used herein, encompasses administration of two or more agents to a subject so that both agents and/or their metabolites are present in the subject at the same time. Co-administration includes simultaneous administration in separate compositions, administration at different times in separate compositions, or administration in a composition in which both agents are present.

Pharmaceutically acceptable forms of the compounds recited herein include pharmaceutically acceptable salts, chelates, non-covalent complexes, prodrugs, and mixtures thereof. In certain embodiments, the compounds described herein are in the form of pharmaceutically acceptable salts. Hence, the terms "chemical entity" and "chemical entities" also encompass pharmaceutically acceptable salts, chelates, non-covalent complexes, prodrugs, and mixtures.

When a composition exists as a mixture of polymorphs, the percentage of each polymorphic component may be determined by one or more techniques well known in the art, including, but not limited to, solid state NMR, IR and XRPD.

The following abbreviations have the following meanings. If an abbreviation is not defined, the generally accepted meaning applies.
- Atm:: atmosphere
- DCC:: dicyclohexylcarbodiimide
- DCM:: dichloromethane
- DIPA:: diisopropylamine
- DMAP:: 4-N,N-dimethylaminopyridine
- DMF:: N,N-dimethylformamide
- DMSO:: dimethylsulfoxide
- g:: gram
- h:: hour
- HPLC:: high pressure liquid chromatography
- L:: liter
- LC/MS:: liquid chromatography/mass spectroscopy
- M:: molar
- Min:: minute
- mL:: milliliter
- mmol:: millimoles
- NHS:: N-hydroxysuccinimide
- TBAB:: tetrabutylamonium bromide
- THF:: tetrahydrofuran
- TFA:: trifluoroacetic acid
- TLC:: thin layer chromatography
- TMS:: trimethylsilyl
- µL:: microliter
- µM:: micromolar
- v/v:: volume to volume

Reference will now be made in detail to preferred embodiments. Although preferred embodiments are described, it will be understood that the invention is not limited to those preferred embodiments. To the contrary, it is intended to cover alternatives, modifications and equivalents as may be included within the spirit and scope of the invention as defined by any claim(s) issuing here from.

### Crystalline forms of the compound of Formula I ((S)-3-((((R)-1-((S)-2-(6-methoxynaphthalen-2-yl)propanoyloxy)ethoxy)carbonylamino)methyl)-5-methylhexanoic acid) and preparation thereof

The chemical entities described herein can generally be synthesized by an appropriate combination of generally well known synthetic methods. Techniques useful in synthesizing these chemical entities are both readily apparent and accessible to those of skill in the relevant art, based on the instant disclosure. Many of the optionally substituted starting compounds and other reactants are commercially available, e.g., from Aldrich Chemical Company (Milwaukee, WI) or can be readily prepared by those skilled in the art using commonly employed synthetic methodology.

The crystalline forms made according to the methods of the invention may be characterized by any methodology according to the art. For example, the crystalline forms made according to the methods of the invention may be characterized by X-ray powder diffraction (XRPD), differential scanning calorimetry (DSC), thermogravimetric analysis (TGA), hot-stage microscopy, and spectroscopy (e.g., Raman, solid state nuclear magnetic resonance (ssNMR), and infrared (IR)).

### XRPD

The crystalline forms according to the invention may be characterized by X-ray powder diffraction patterns (XRPD). The relative intensities of XRPD peaks can vary, depending upon the particle size, the sample preparation technique, the sample mounting procedure and the particular instrument employed. Moreover, instrument variation and other factors can affect the 2-θ values. Therefore, the XRPD peak assignments can vary by plus or minus about 0.3 degrees.

### DSC

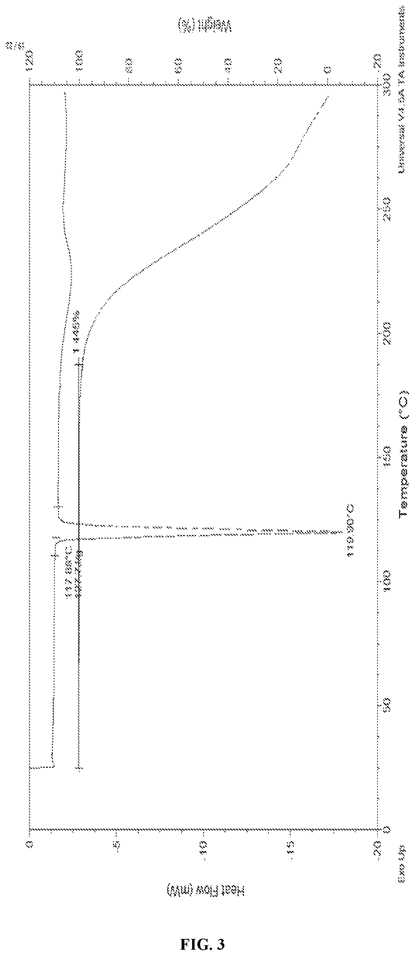

The crystalline forms according to the invention can also be identified by its characteristic differential scanning calorimeter (DSC) trace such as shown in FIG. 3. For DSC, it is known that the temperatures observed will depend upon the rate of temperature change as well as sample preparation technique and the particular instalment employed. Thus, the values reported herein relating to DSC thermograms can vary by plus or minus about 4 °C.

### TGA

The crystalline forms of the invention may also give rise to thermal behavior different from that of the amorphous material or another polymorphic form. Thermal behavior may be measured in the laboratory by thermogravimetric analysis (TGA) which may be used to distinguish some polymorphic forms from others. In one aspect, the crystalline forms may be characterized by thermogravimetric analysis.

The crystalline forms of the invention are useful in the production of medicinal preparations and can be obtained by means of a crystallization process to produce crystalline and semi-crystalline forms or a solidification process to obtain the amorphous form. In various embodiments, the crystallization is carried out by either generating the compound of Formula I in a reaction mixture and isolating the desired polymorph from the reaction mixture, or by dissolving raw compound in a solvent, optionally with heat, followed by crystallizing/solidifying the product by cooling (including active cooling) and/or by the addition of an anti-solvent for a period of time. The crystallization or solidification may be followed by drying carried out under controlled conditions until the desired water and/or solvent content is reached in the end polymorphic form.

Exemplary crystalline forms of the compound of Formula I and methods of making the same are disclosed in detail herein. It should be understood that methods described herein also apply to the stereoisomers or Formula I, as well as to its isotopically labeled derivatives (e.g., ²H, ³H, ¹³C, ¹⁴C, ¹⁵N, ¹⁷O, ¹⁸O, etc.).

In one aspect, the invention is directed to methods of making crystalline forms of the compound of the Formula I or a pharmaceutically acceptable salt and/or solvate thereof by recrystallization of the compound of Formula I. The compound of Formula I can be synthesized by any applicable method. For example, the compound Formula I can be prepared according to the methods described in Feng Xu, International Publication No. WO 2010/042759, which is incorporated herein by reference in its entirety. Further, exemplary methods of preparation of the compound of Formula I are described in Examples 1 and 2 below. Isolation and purification of the compound of Formula I can be effected, if desired, by any suitable separation or purification procedure such as, for example, filtration, extraction, crystallization, column chromatography, thin-layer chromatography or thick-layer chromatography, or a combination of these procedures. Specific illustrations of suitable separation and isolation procedures are detailed in Examples 1 and 2 below. However, other known separation or isolation procedures can also be used.

In some embodiments, the compound of Formula I as prepared by these methods is isolated as an off-white or white solid after lyophilization from aqueous acetonitrile. The compound of Formula I obtained by these methods may be partially or wholly amorphous. Further, the compound of Formula I obtained by these methods may certain alkali metal salt forms which may be hygroscopic.

The amorphous solids are difficult to handle under pharmaceutical processing conditions because of low bulk densities. Moreover, handling of hygroscopic solids further requires special techniques and equipment. Furthermore, drugs that are hygroscopic must be packaged in special containers that have water vapor barriers, which substantially increases the cost of such products. Accordingly, a need exists for crystalline forms of Formula I with superior physicochemical properties that may be used advantageously in pharmaceutical processing and pharmaceutical compositions.

Prior to crystallization, the compound of Formula I may be isolated with a chemical purity in the range of about 50-100%, for example about 60%-100%, about 70%-100%, about 80%-100%, or about 90%-100%. In some embodiments, prior to crystallization, the compound of Formula I is isolated with a chemical purity greater than 90% , greater than 91% , greater than 92%, greater than 93%, greater than 94% , greater than 95% , greater than 96% , greater than 97%, greater than 98%, or greater than 99% . In some embodiments, prior to crystallization, the compound of Formula I is isolated with a chemical purity approaching 100%.

In some embodiments, crystalline forms of Formula I are prepared by recrystallization of the compound of Formula I. In some embodiments, the recrystallization comprises adding the compound of Formula I to a solvent to form a solution or suspension. As used in the disclosure, the terms solution and suspension are used interchangeably and are meant to include circumstances where the compound of Formula I is placed in a solvent or solvent mixture regardless of solubility. The solvent used in recrystallization may be a homogenous solvent, a combination of solvents, or a solvent or solvent combination in which the compound of Formula I exhibits temperature dependent solubility. In some embodiments, the solvent used for recrystallization is a solvent or a solvent combination in which the compound of Formula I is soluble within a first temperature range, and poorly soluble within a second temperature range. In some embodiments, the solvent used for recrystallization is a mixture of a "good" solvent and an "anti-solvent."

Non-limiting examples of suitable "good" solvents include organic alcohols (for example methanol, ethanol, 1,2-propanediol, t-butanol, n-butanol, isopropanol), acetic acid, nitromethane, acetonitrile, dimethylsulfoxide, dimethylformamide, N-methylpyrrolidone, acetone, methyl acetate, ethyl acetate, isopropyl acetate, isobutyl acetate, methyl isobutyl ketone, 1,2 dimethoxyethane, tetrahydrofuran, 2-methyl tetrahydrofuran, toluene, methyl t-butyl ether, chlorobenzene, 1,4-dioxane, diethylether, cumene, o-xylene, m-xylene, p-xylene, 2-ethoxyethanol, 1,2-ethandiol, ethyl formate, 2-methoxyethanol, 1-pentanol, anisole, dichloromethane, cis and trans 1,2-dichloroethylene, chloroform, dimethylacetamide, propyl acetate and mixtures thereof.

Non-limiting of suitable "anti-solvents" include alkanes such as pentane, hexane, heptane, octane, nonane, decane, undecane, dodecane, cis- or trans-decalin, cyclohexane, methylcyclohexane and mixtures thereof.

In some embodiments, the dissolution process is carried out at elevated temperature. In some examples, the dissolving of the compound of Formula I in the recrystallization solvent system is performed at a temperature of about 40-90 °C, 50-90 °C, 60-90 °C, 70-90 °C, 80-90 °C, 40-80 °C, 50-80 °C, 60-80 °C, 70-80 °C, 40-70 °C, 50-70 °C, 60-70 °C, 40-60 °C, 50-60 °C, or 40-50 °C. In some examples, the temperature for dissolution is a temperature in the range of from about room temperature to up to and including the boiling point of the recrystallization solvent. Accordingly, in some embodiments, the compound of Formula I is dissolved in a solvent or solvent mixture with heating and optionally, with shaking or stirring. The heated solution may be kept at elevated temperature to ensure complete dissolution of the compound of Formula I. The heated solution may also be filtered at elevated temperature to remove any undissolved components. In some embodiments, the post dissolution solution is cooled slowly to provide crystalline from of Formula I, which may be separated from residual solvent by filtration and/or drying. In some embodiments, the solution is cooled to a temperature of from about - 25 °C to about 25 °C, for example from about -15 °C-about 25 °C, from about 0 °C to about 25 °C, from about -15 °C to about 15 °C, or from about -5°C to about 5°C. In some embodiments, the solution is cooled to a temperature of about 0-30 °C, 5-30 °C, 10-30 °C, 15-30 °C, 20-30 °C, 25-30 °C, 0-20 °C, 5-20 °C, 10-20 , 15-20 °C, 18-20 °C, 0-10 °C, 5-10 °C, or 0-5 °C

In some embodiments, the obtained crystalline forms of the compound of Formula I can further be dried. Drying can be conducted at atmospheric pressure or under reduced pressure, at atmospheric temperature or at elevated temperature.

Other methods, known to those of skill in the crystallization arts, (e.g., solvent evaporation, drowning-out, chemical reaction, seeding with a small quantity of the desired crystal form, etc.) may also be employed to provide crystalline forms of the compound of Formula I.

In some embodiments, the recrystallization of the compound of Formula I involves dissolving the compound of Formula I in a solvent comprising isopropanol (for example, isopropanol). In some embodiments, the ratio of the amount of the compound of Formula I dissolved to the amount of solvent used is in the range of from about 10:1 to about 1:10 w/v, for example from about 9:1 to about 1:10, 8:1 to about 1:10, 7: 1 to about 1:10, 6:1 to about 1:10, 5:1 to about 1:10, 4:1 to about 1:10, 3:1 to about 1:10, 2:1 to about 1:10, 1:2 to about 1:10, 1:3 to about 1:10, 1:4 to about 1:10, 1:5 to about 1:10, 1:6 to about 1:10, 1:7 to about 1:10, 1:8 to about 1:10, or 1:9 to about 1:10 w/v. In some embodiments, the ratio of the amount of the compound of Formula I dissolved to the amount of solvent used is about 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, or 1:10 w/v.

In some embodiments, the ratio of the amount of the compound of Formula I dissolved to the amount of solvent used is about 1:2 w/ v. In some examples, the dissolving of the compound of Formula I in the solvent comprising isopropanol is carried at a temperature between about 20 °C and about reflux temperature, for example, at about 20 °C-80 °C, about 20 °C-70 °C, about 20 °C-60 °C, about 20 °C-50 °C, about 20 °C-40 °C, about 20 °C-30 °C, about 30 °C-80 °C, about 30 °C-70 °C, about 30 °C-60 °C, about 30 °C-50 °C, about 30 °C-40 °C, about 40 °C-80 °C about 40 °C-70 °C, about 40 °C-60 °C, about 40 °C-50 °C, about 50 °C-80 °C, about 50 °C-70 °C, about 50 °C-60 °C, about 60 °C-80 °C, about 60 °C-70 °C, or about 70 °C-80 °C. In some examples, the dissolving of the compound of Formula I in the solvent comprising isopropanol is carried at a temperature of about 40 °C, for example at a temperature of about 30 °C -50 °C or about 35 °C -45 °C.

In some embodiments, the recrystallization of the compound of Formula I further involves addition of an anti-solvent to the solution of the compound of Formula I in the solvent comprising isopropanol. In some embodiments, the anti-solvent is an alkane, for example heptane. In some embodiments, the ratio of the amount of heptane added to the amount of the solvent comprising isopropanol is between about 10:1 to about 1:1 v/v, for example between 9:1-8:1, 9:1-7:1, 9:1-6:1, 9:1-5:1, 9:1-4:1, 9:1-3:1, 9:1-2:1, 9:1-1:1, 8:1-7:1, 8:1-6:1, 8:1-5:1, 8:1-4:1, 8:1-3:1, 8:1-2:1, 8:1-1:1, 7:1-6:1, 7:1-5:1, 7:1-4:1, 7:1-3:1, 7:1-2:1, 7:1-1:1, 6:1-5:1, 6:1-4:1, 6:1-3:1, 6:1-2:1, 6:1-1:1, 5:1-4:1, 5:1-3:1, 5:1-2:1, 5:1-1:1, 4:1-3:1, 4:1-2:1, 4:1-1:1, 3:1-2:1, 3:1-1:1, or 2:1-1:1 v/v. In some embodiments, the ratio of the amount of heptane added to the amount of the solvent comprising isopropanol is between about 6:1 to about 5:1 v/v, for example about 5.5:1 v/v.

In some embodiments, the anti-solvent (for example heptane) is added slowly (for example dropwise) over a period of time. In some embodiments, the anti-solvent is added over a period of about 5 hours to about 1 min, for example about 4.5 h-1 min, 4 h-1 min, 3.5 h-1 min, 3 h-1 min, 2.5 h-1 min, 2 h-1 min, 1.5 h-1 min, 1 h-1 min, 45 min-1 min, 30 min-1 min, 15 min-1 min, 5 min-1 min, 5 h-15 min 4.5 h-15 min, 4 h-15 min, 3.5 h-15 min, 3 h-15 min, 2.5 h-15 min, 2 h-15 min, 1.5 h-15 min, 1 h-15 min, 45 min-15 min, 30 min-15 min, 5 h-30 min, 4.5 h-30 min, 4 h-30 min, 3.5 h-30 min, 3 h-30 min, 2.5 h-30 min, 2 h-30 min, 1.5 h-30 min, 1 h-30 min, 45 min-30 min, 5 h-45 min, 4.5 h-45 min, 4 h-45 min, 3.5 h-45 min, 3 h-45 min, 2.5 h-45 min, 2 h-45 min, 1.5 h-45 min, 1 h-45 min, 5 h-1 h, 4.5 h-1 h, 4 h-1 h, 3.5 h-1 h, 3 h-1 h, 2.5 h-1 h, 2 h-1 h, 1.5 h-1 h, 5 h-2 h, 4.5 h-2 h, 4 h-2 h, 3.5 h-2 h, 3 h-2 h, 2.5 h-2 h, 5 h-2.5 h, 4.5 h-2.5 h, 4 h-2.5 h, 3.5 h-2.5 h, 3 h-2.5 h, 5 h-3 h, 4.5 h-3 h, 4 h-3 h, 3.5 h-3h, 5 h-3.5 h, 4.5 h-3.5 h, 4 h-3.5 h, 5 h-4 h, 4.5 h-4 h, 05 5h-4.5 h. In some embodiments, the anti-solvent is added over a period of about 2 h-about 5 min, for example over a period of about 1 h.

In some embodiments, the recrystallization of the compound of Formula I further involves cooling the solution of the compound of Formula I after addition of the anti-solvent (for example heptane). In some embodiments, the solution is cooled to a temperature of about 0 °C to about 30 °C. In some embodiments, the solution is cooled to a temperature of at least about 0 °C. In some embodiments, the solution is cooled to a temperature of at most about 30 °C. In some embodiments, the solution is cooled to a temperature of about 0 °C to about 5 °C, about 0 °C to about 10 °C, about 0 °C to about 15 °C, about 0 °C to about 20 °C, about 0 °C to about 25 °C, about 0 °C to about 30 °C, about 5 °C to about 10 °C, about 5 °C to about 15 °C, about 5 °C to about 20 °C, about 5 °C to about 25 °C, about 5 °C to about 30 °C, about 10 °C to about 15 °C, about 10 °C to about 20 °C, about 10 °C to about 25 °C, about 10 °C to about 30 °C, about 15 °C to about 20 °C, about 15 °C to about 25 °C, about 15 °C to about 30 °C, about 20 °C to about 25 °C, about 20 °C to about 30 °C, or about 25 °C to about 30 °C. In some embodiments, the solution is cooled to a temperature of about 0 °C, about 5 °C, about 10 °C, about 15 °C, about 20 °C, about 25 °C, or about 30 °C. In some embodiments, the solution is cooled to a temperature of about 15 °C to about 25 °C, or example about 20 °C.

In some embodiments, the solution is maintained at this temperature for about 5 hours to about 1 min, for example about 4.5 h-1 min, 4 h-1 min, 3.5 h-1 min, 3 h-1 min, 2.5 h-1 min, 2 h-1 min, 1.5 h-1 min, 1 h-1 min, 45 min-1 min, 30 min-1 min, 15 min-1 min, 5 min-1 min, 5 h-15 min 4.5 h-15 min, 4 h-15 min, 3.5 h-15 min, 3 h-15 min, 2.5 h-15 min, 2 h-15 min, 1.5 h-15 min, 1 h-15 min, 45 min-15 min, 30 min-15 min, 5 h-30 min, 4.5 h-30 min, 4 h-30 min, 3.5 h-30 min, 3 h-30 min, 2.5 h-30 min, 2 h-30 min, 1.5 h-30 min, 1 h-30 min, 45 min-30 min, 5 h-45 min, 4.5 h-45 min, 4 h-45 min, 3.5 h-45 min, 3 h-45 min, 2.5 h-45 min, 2 h-45 min, 1.5 h-45 min, 1 h-45 min, 5 h-1 h, 4.5 h-1 h, 4 h-1 h, 3.5 h-1 h, 3 h-1 h, 2.5 h-1 h, 2 h-1 h, 1.5 h-1 h, 5 h-2 h, 4.5 h-2 h, 4 h-2 h, 3.5 h-2 h, 3 h-2 h, 2.5 h-2 h, 5 h-2.5 h, 4.5 h-2.5 h, 4 h-2.5 h, 3.5 h-2.5 h, 3 h-2.5 h, 5 h-3 h, 4.5 h-3 h, 4 h-3 h, 3.5 h-3h, 5 h-3.5 h, 4.5 h-3.5 h, 4 h-3.5 h, 5 h-4 h, 4.5 h-4 h, 5h-4.5 h. In some embodiments, the solution is maintained at this temperature for about greater than 5 hours, for example for about 6 h, 7 h, 8 h, 9 h, 10 h, 11h, 12 h, 13 h, 14 h, 15 h, 16 h, 17 h, 18 h, 19 h, or 20 h. In some embodiments, the solution is maintained at this temperature for about 1 h. In some embodiments, the solution is cooled to a temperature of about 15 °C to about 25 °C, or example about 20 °C and maintained at this temperature for about 1 h. In some embodiments, the solution is cooled to a first lower temperature, maintained at that temperature for a first period of time, then cooled to a second temperature and maintained at the second lowered temperature for a second period of time. In some embodiments the solution is cooled to a temperature of about 15 °C to about 25 °C, or example about 20 °C and maintained at this temperature for about 1 h and then cooled to about 0-10 °C and maintained at that temperature for about 15 hours.

In some embodiments, the recrystallization of the compound of Formula I further involves filtering and/or washing the obtained crystalline form. Washing can be performed by any suitable solvent, for example by a solvent comprising isopropanol, heptane or a mixture thereof. In some embodiments, the obtained crystalline form is washed with heptane.

In some embodiments, the recrystallization of the compound of Formula I comprises dissolving the compound of Formula I in a solvent comprising ethyl acetate, heptane or a mixture thereof. In some embodiments, the ratio of the amount of heptane to ethyl acetate in the solvent is about 20:1 to about 1:20 v/v, for example, the ratio of the amount of heptane to ethyl acetate in the solvent is about 20 to about 1. In some embodiments, the ratio of the amount of heptane to ethyl acetate in the solvent is at least about 1. In some embodiments, the ratio of the amount of heptane to ethyl acetate in the solvent is at most about 20. In some embodiments, the ratio of the amount of heptane to ethyl acetate in the solvent is about 20 to about 15, about 20 to about 10, about 20 to about 5, about 20 to about 1, about 15 to about 10, about 15 to about 5, about 15 to about 1, about 10 to about 5, about 10 to about 1, or about 5 to about 1. In some embodiments, the ratio of the amount of heptane to ethyl acetate in the solvent is about 20:1, about 15: 1, about 10: 1, about 5:1, about 1:1, about 1:5, about 1: 10, about 1:15, or about 1:20. In some embodiments, the ratio of the amount of heptane to the amount of heptane in the solvent is about 10:1 by v/v. In some embodiments, the dissolution of the compound of Formula I in the solvent comprising heptane and ethyl acetate is done at a temperature above the ambient temperature, for example at a temperature between about 50 °C and about the reflux temperature. In some embodiments, dissolution is carried out at a temperature of about 50 °C-80°C, about 60 °C-80 °C, about 70 °C-80 °C, about 50 °C -70 °C, about 60 °C-70 °C, or about 50 °C-60 °C. In some embodiments, dissolution is carried at a temperature of about 70° C.

In some embodiments, the recrystallization of the compound of Formula I comprises cooling the solution of the compound of Formula I in heptane and ethyl acetate to a temperature of about 0 °C to about 30 °C. In some embodiments, the recrystallization of the compound of Formula I comprises cooling the solution of the compound of Formula I in heptane and ethyl acetate to a temperature of about at least about 0 °C. In some embodiments, the recrystallization of the compound of Formula I comprises cooling the solution of the compound of Formula I in heptane and ethyl acetate to a temperature of about at most about 30 °C. In some embodiments, the recrystallization of the compound of Formula I comprises cooling the solution of the compound of Formula I in heptane and ethyl acetate to a temperature of about 0 °C to about 5 °C, about 0 °C to about 10 °C, about 0 °C to about 15 °C, about 0 °C to about 20 °C, about 0 °C to about 25 °C, about 0 °C to about 30 °C, about 5 °C to about 10 °C, about 5 °C to about 15 °C, about 5 °C to about 20 °C, about 5 °C to about 25 °C, about 5 °C to about 30 °C, about 10 °C to about 15 °C, about 10 °C to about 20 °C, about 10 °C to about 25 °C, about 10 °C to about 30 °C, about 15 °C to about 20 °C, about 15 °C to about 25 °C, about 15 °C to about 30 °C, about 20 °C to about 25 °C, about 20 °C to about 30 °C, or about 25 °C to about 30 °C. In some embodiments, the recrystallization of the compound of Formula I comprises cooling the solution of the compound of Formula I in heptane and ethyl acetate to a temperature of about 0 °C, about 5 °C, about 10 °C, about 15 °C, about 20 °C, about 25 °C, or about 30 °C.

In some embodiments, the recrystallization of the compound of Formula I further involves filtering and/or washing the obtained crystalline form. Washing can be performed by any suitable solvent, for example by a solvent comprising ethyl acetate and/or heptane.

In some embodiments, the recrystallization of the compound of Formula I involves dissolving the compound of Formula I in a solvent comprising methylcyclohexane and/or methyl t-butyl ether. In some embodiments, the recrystallization of the compound of Formula I involves dissolving the compound of Formula I in a mixture of methylcyclohexane and methyl t-butyl ether. The ratio of the amount of methylcyclohexane to methyl t-butyl ether can be between about 1:1 to about 30:1 v/v. In some embodiments, the ratio of the amount of methylcyclohexane to methyl t-butyl ether can be at least about 1. In some embodiments, the ratio of the amount of methylcyclohexane to methyl t-butyl ether can be at most about 30. In some embodiments, the ratio of the amount of methylcyclohexane to methyl t-butyl ether can be about 1 to about 5, about 1 to about 10, about 1 to about 15, about 1 to about 20, about 1 to about 25, about 1 to about 30, about 5 to about 10, about 5 to about 15, about 5 to about 20, about 5 to about 25, about 5 to about 30, about 10 to about 15, about 10 to about 20, about 10 to about 25, about 10 to about 30, about 15 to about 20, about 15 to about 25, about 15 to about 30, about 20 to about 25, about 20 to about 30, or about 25 to about 30. In some embodiments, the ratio of the amount of methylcyclohexane to methyl t-butyl ether can be about 1, about 5, about 10, about 15, about 20, about 25, or about 30.

The dissolution of the compound of Formula I in the solvent comprising methylcyclohexane and/or methyl t-butyl ether is carried out at a temperature between of about 10 °C and about 60 °C. In some embodiments, dissolution is carried out at a temperature of about at least about 10 °C. In some embodiments, dissolution is carried out at a temperature of about at most about 60 °C. In some embodiments, dissolution is carried out at a temperature of about 10 °C to about 15 °C, about 10 °C to about 20 °C, about 10 °C to about 25 °C, about 10 °C to about 30 °C, about 10 °C to about 35 °C, about 10 °C to about 40 °C, about 10 °C to about 45 °C, about 10 °C to about 50 °C, about 10 °C to about 55 °C, about 10 °C to about 60 °C, about 15 °C to about 20 °C, about 15 °C to about 25 °C, about 15 °C to about 30 °C, about 15 °C to about 35 °C, about 15 °C to about 40 °C, about 15 °C to about 45 °C, about 15 °C to about 50 °C, about 15 °C to about 55 °C, about 15 °C to about 60 °C, about 20 °C to about 25 °C, about 20 °C to about 30 °C, about 20 °C to about 35 °C, about 20 °C to about 40 °C, about 20 °C to about 45 °C, about 20 °C to about 50 °C, about 20 °C to about 55 °C, about 20 °C to about 60 °C, about 25 °C to about 30 °C, about 25 °C to about 35 °C, about 25 °C to about 40 °C, about 25 °C to about 45 °C, about 25 °C to about 50 °C, about 25 °C to about 55 °C, about 25 °C to about 60 °C, about 30 °C to about 35 °C, about 30 °C to about 40 °C, about 30 °C to about 45 °C, about 30 °C to about 50 °C, about 30 °C to about 55 °C, about 30 °C to about 60 °C, about 35 °C to about 40 °C, about 35 °C to about 45 °C, about 35 °C to about 50 °C, about 35 °C to about 55 °C, about 35 °C to about 60 °C, about 40 °C to about 45 °C, about 40 °C to about 50 °C, about 40 °C to about 55 °C, about 40 °C to about 60 °C, about 45 °C to about 50 °C, about 45 °C to about 55 °C, about 45 °C to about 60 °C, about 50 °C to about 55 °C, about 50 °C to about 60 °C, or about 55 °C to about 60 °C. In some embodiments, dissolution is carried out at a temperature of about 10 °C, about 15 °C, about 20 °C, about 25 °C, about 30 °C, about 35 °C, about 40 °C, about 45 °C, about 50 °C, about 55 °C, or about 60 °C. In some embodiments, dissolution is carried out at a temperature of about 20 °C to about 40 °C.

In some embodiments, the recrystallization of the compound of Formula I further comprises cooling the solution of the compound of Formula I in methylcyclohexane and/or methyl t-butyl ether further comprises cooling the solution to a temperature of about 0° C. to about 30 °C. In some embodiments, the solution is cooled to a temperature of about at least about 0 °C. In some embodiments, the solution is cooled to a temperature of about at most about 30 °C. In some embodiments, the solution is cooled to a temperature of about 0 °C to about 5 °C, about 0 °C to about 10 °C, about 0 °C to about 15 °C, about 0 °C to about 20 °C, about 0 °C to about 25 °C, about 0 °C to about 30 °C, about 5 °C to about 10 °C, about 5 °C to about 15 °C, about 5 °C to about 20 °C, about 5 °C to about 25 °C, about 5 °C to about 30 °C, about 10 °C to about 15 °C, about 10 °C to about 20 °C, about 10 °C to about 25 °C, about 10 °C to about 30 °C, about 15 °C to about 20 °C, about 15 °C to about 25 °C, about 15 °C to about 30 °C, about 20 °C to about 25 °C, about 20 °C to about 30 °C, or about 25 °C to about 30 °C. In some embodiments, the solution is cooled to a temperature of about 0 °C, about 5 °C, about 10 °C, about 15 °C, about 20 °C, about 25 °C, or about 30 °C. In some embodiments, the solution is cooled to a temperature of about 0° C. to about 25 ° C to provide crystalline (S)-3-((((R)-1-((S)-2-(6-methoxynaphthalen-2-yl)propanoyloxy)ethoxy)carbonyl-amino)methyl)-5-methylhexanoic acid .

In some embodiments, the recrystallization of the compound of Formula I further involves filtering and/or washing the obtained crystalline form. Washing can be performed by any suitable solvent, for example by a solvent comprising methylcyclohexane and/or methyl t-butyl ether.

In some embodiments, the crystalline form of the compound of Formula I, obtained by the methods disclosed herein, has characteristic absorption peaks at 8.6°±0.3°, 15.5°±0.3°, 16.4°±0.3°, 17.9°±0.3°, and 20.6°±0.3°. In some embodiments, the crystalline form of the Formula I has at least one additional characteristic absorption peaks selected from the group consisting of peaks at 6.4°±0.3°, 9.3°±0.3°, 9.8°±0.3°, 17.2°±0.3°, 17.5°±0.3°, 19.9°±0.3°, 20.4°±0.3°, 21.4°±0.3°, 21.7°±0.3°, 22.4°±0.3°, 22.9°±0.3°, 23.7°±0.3°, 23.9°±0.3°, 24.3°±0.3°, 25.0°±0.3°, 25.6°±0.3°, 26.4°±0.3°, 26.9°±0.3°, 27.6°±0.3°, 29.7°±0.3° and 31.3°±0.3°. In some embodiments, the crystalline form of the Formula I has characteristic absorption peaks at 6.4°±0.3°, 8.6°±0.3°, 9.3°±0.3°, 9.8°±0.3°, 15.5°±0.3°, 16.4°±0.3°, 17.2°±0.3°, 17.5°±0.3°, 17.9°±0.3°, 19.9°±0.3°, 20.4°±0.3°, 20.6°±0.3°, 21.4°±0.3°, 21.7°±0.3°, 22.4°±0.3°, 22.9°±0.3°, 23.7°±0.3°, 23.9°±0.3°, 24.3°±0.3°, 25.0°±0.3°, 25.6°±0.3°, 26.4°±0.3°, 26.9°±0.3°, 27.6°±0.3°, 29.7°±0.3° and 31.3°±0.3°.

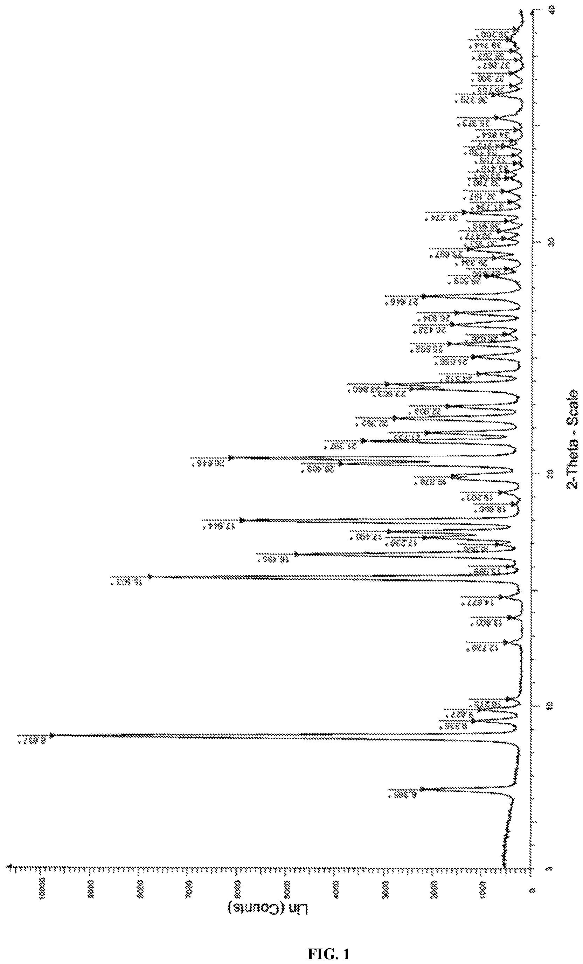

In some embodiments, the crystalline form of the Formula I obtained by the methods disclosed herein has characteristic absorption peaks at 8.6°±0.3°, 15.5°±0.3°, 16.4°±0.3°, 17.9°±0.3°, and 20.6°±0.3° in an X-ray powder diffractogram. In some embodiments, the X-ray powder diffractogram of the crystalline form of Formula I has at least one additional characteristic absorption peaks selected from the group consisting of peaks at 6.4°±0.3°, 9.3°±0.3°, 9.8°±0.3°, 17.2°±0.3°, 17.5°±0.3°, 19.9°±0.3°, 20.4°±0.3°, 21.4°±0.3°, 21.7°±0.3°, 22.4°±0.3°, 22.9°±0.3°, 23.7°±0.3°, 23.9°±0.3°, 24.3°±0.3°, 25.0°±0.3°, 25.6°±0.3°, 26.4°±0.3°, 26.9°±0.3°, 27.6°±0.3°, 29.7°±0.3° and 31.3°±0.3°. In other embodiments, the crystalline form of Formula has characteristic absorption peaks at 6.4°±0.3°, 8.6°±0.3°, 9.3°±0.3°, 9.8°±0.3°, 15.5°±0.3°, 16.4°±0.3°, 17.2°±0.3°, 17.5°±0.3°, 17.9°±0.3°, 19.9°±0.3°, 20.4°±0.3°, 20.6°±0.3°, 21.4°±0.3°, 21.7°±0.3°, 22.4°±0.3°, 22.9°±0.3°, 23.7°±0.3°, 23.9°±0.3°, 24.3°±0.3°, 25.0°±0.3°, 25.6°±0.3°, 26.4°±0.3°, 26.9°±0.3°, 27.6°±0.3°, 29.7°±0.3° and 31.3°±0.3° in an X-ray powder diffractogram. In some embodiments, the X-ray powder diffractogram of the crystalline form of Formula I obtained by the methods disclosed herein is essentially identical to the X-ray powder diffractogram shown in FIG. 1.

In some embodiments, the crystalline form of Formula I has a melting point in the range of from about 100° C to about 140° C. In some embodiments, the crystalline form of Formula I has a melting point in the range of from about 110 °C to about 130 °C. In some embodiments, the crystalline form of Formula I has a melting point in the range of from about 115 °C to about 125 °C. In some embodiments, the crystalline from of Formula I has a melting point in the range of from about 117 °C to about 123 °C, for example in the range of from about 118 °C to about 122 °C. In some embodiments, the crystalline form of Formula I has a melting point in the range of from about 119 °C to about 121 °C.

### Therapeutic Uses

The crystalline forms of Formula I and/or pharmaceutical compositions thereof may be used for treatment of a disease in a subject in need thereof. A suitable subject can be, e.g., a human, a non-human primate (including but not limited to a gorilla, chimpanzee, orangutan, or a monkey), a rodent (including but not limited to a mouse, rat, guinea pig, or gerbil) a dog, a cat, horse, cow, pig, sheep, rabbit, or goat. In some embodiments, the subject is a mammal, for example a human.

In some embodiments, the crystalline forms of the invention are administered to a mammal, preferably a human, to treat a neurological disorder, epilepsy, or pain including but not limited to centrally mediated pain, peripherally mediated pain, structural or soft tissue injury related pain, progressive disease related pain (i.e., oncology), musculoskeletal pain, and a neuropathic pain state, all of which includes acute (i.e., acute injury or trauma, pre and post-surgical, headache such as a migraine), chronic (i.e., neuropathic pain conditions such diabetic peripheral neuropathy and post-herpatic neuralgia) and inflammatory condition (i.e., osteo or rheumatoid arthritis, sequela to acute injury or trauma) pain states, depression, anxiety, psychosis, faintness attacks, hypokinesia, cranial disorders, neurodegenerative disorders, panic, insomnia, gastrointestinal disorders, addictive disorders (e.g. ethanol, cocaine), restless leg syndrome.

In other embodiments, the crystalline forms of the invention are administered to an animal, for example a human, as a preventative/prophylactic measure against various disorders including predisposition for a neurological disorder, epilepsy, pain including but not limited to centrally mediated pain, peripherally mediated pain, structural or soft tissue injury related pain, progressive disease related pain (i.e., oncology), musculoskeletal pain, and neuropathic pain states, all of which would include acute (i.e., acute injury or trauma, pre and post-surgical, headache such as a migraine), chronic (i.e., neuropathic pain conditions such diabetic peripheral neuropathy and post-herpatic neuralgia) and inflammatory condition (i.e., osteo or rheumatoid arthritis, sequela to acute injury or trauma) pain states, depression, anxiety, psychosis, faintness attacks, hypokinesia, cranial disorders, neurodegenerative disorders, panic, insomnia, gastrointestinal disorders, addictive disorders (e.g. ethanol, cocaine), and restless leg syndrome.

In further embodiments, the crystalline forms of the invention are used for the prevention of one disorder and concurrently for the treatment of another disorder mentioned hereinabove, for example, the crystalline forms can be used for the prevention of psychosis or addiction and the treatment of pain. Or for example, the crystalline forms can be used for the prevention of pain and for the treatment of an inflammatory disease. Or for example, the crystalline forms can be used for the prevention of an inflammatory disease and for the treatment of a pain.

The suitability of the crystalline forms of the invention or of the pharmaceutical compositions thereof in treating pain (e.g., neuropathic pain and musculoskeletal pain), inflammatory disease (e.g. arthritis) may be determined by methods known in the art. Exemplary methods can be found for e.g. in U.S. Pat. No. 5,563,175, U.S. Pat. No. 6,001,876, U.S. Pat. No. 6,028,214, U.S. Pat. No. 6,117,906, U.S. Pat. No. 8,268,887, International Application Publication No. WO 1992/09560, International Application Publication No. WO 1993/23383, International Application Publication No. WO 1997/29101, International Application Publication No. 1997/33858, International Application Publication No. 1997/33859, International Application Publication No. WO 1998/ 17627, International Application Publication No. WO 1999/08671, International Application Publication No. WO 1999/21824, International Application Publication No. WO 1999/31057, International Application Publication No. WO 1999/37296, International Application Publication No. WO 1999/31075, International Application Publication No. WO 1999/ 61424, International Application Publication No. WO 2000/23067, International Publication No. WO 2000/31020, International Publication No. WO 2000/50027, and International Publication No. WO 2002/ 00209, each of which is incorporated herein by reference in its entirety.

The crystalline forms of the invention and/or pharmaceutical compositions thereof may be used to treat or prevent pain (e.g. neuropathic pain and musculoskeletal pain) and/or inflammatory disease (e.g. arthritis) using known procedures described in the art. In some embodiments, the crystalline forms disclosed herein, may be more efficacious than the corresponding non-crystalline form and/or parent drug molecules (pregabalin and/or naproxen) in treating or preventing pain (e.g. neuropathic pain and musculoskeletal pain) or inflammatory disease (e.g. arthritis) because the disclosed crystalline forms release both naproxen and pregabalin in *vivo.*

In some embodiments, it may require less quantity for each parent drug to reach the therapeutic concentrations in the blood, i.e. without wishing to bound by theory, it is believed that the crystalline forms of the compound of Formula I disclosed herein are stable before they are absorbed from the gastrointestinal lumen into the blood, and then they are hydrolyzed to release naproxen and pregabalin in the blood. Therefore, the crystalline forms of the compounds of Formula I disclosed herein have less GI toxicity compared to naproxen when taken orally. For example, NSAIDs, such as naproxen is believed to inhibit both the COX-1 and COX-2 enzymes non-selectively. This results in the inhibition of prostaglandin synthesis and leukocyte activation. Prostaglandins act as the signaling molecules in the body, inducing inflammation. Thus, by inhibiting COX-1/2, naproxen induces an anti-inflammatory and analgesic effect. The primary reason for non-specific NSAID-induced GI injury is proposed due to its disruption of the mucosal barrier by reducing the production of bicarbonate, prostaglandins, directly damaging the gastric mucosal epithelium and impairing the repair processes. The injury is the net effect of the systemic and topical effects of NSAIDs on the upper GI system. However, many studies suggested that the local exposure of the high NSAIDs, such as naproxen concentration plays a critical role in causing the GI injury (e.g., ulcer or bleeding) comparing to systemic exposure (Banoob, D. W.; et al., Ann Pharmacother. 2002, 36(1), 163-6; Petroski, D.; Clin Ther. 1993, 15(2), 314-20; Van, G. A.; et al., Dig. Dis. Sci. 1993, 38(11), 1970-7; D'Haens, G.; et al., J. Clin. Gastroenterol. 1993, 17(3), 207-12. 34. Ivey, K. J.; et al., Dig Dis Sci., 1980, 25(2), 97-99. Interestingly, by "masking" the NSAIDs' biological activity during the absorption process using prodrug strategy, the GI injury can be minimized significantly (Ruchita, S.; et al, Curr Drug Deliv., 2017, 14(1), 16-26). In comparison to naproxen, the crystalline forms disclosed herein, are absorbed across the gut wall along the gastrointestinal tract intact, therefore, the GI side effects induced by naproxen can be reduced significantly.

The crystalline forms of the compound of Formula I may also be more efficacious than naproxen and pregabalin monotherapy in treating or preventing pain (e.g., neuropathic pain and muscular and skeletal pain), post-herpetic neuralgia, inflammatory disease (e.g. arthritis) (Haruhiko N.; et al., J. Med. Sci., 2012, 28, 59-68; Seiji Ohtori, at el. Yonsei Med. J. 2013, 54(5), 1253-1258; Carlo Luca Romano, et al, J. Orthopaed Traumatol, 2009, 10, 185-191).

### Pharmaceutical Compositions

The subject pharmaceutical compositions are typically formulated to provide a therapeutically effective amount of a crystalline from of the present invention as the active ingredient, or a pharmaceutically acceptable salt, ester, prodrug, solvate, hydrate or derivative thereof. Where desired, the pharmaceutical compositions contain the crystalline forms disclosed herein or pharmaceutically acceptable salt and/or coordination complex thereof, and one or more pharmaceutically acceptable excipients, carriers, including inert solid diluents and fillers, diluents, including sterile aqueous solution and various organic solvents, permeation enhancers, solubilizers and adjuvants.

The subject pharmaceutical compositions can be administered alone or in combination with one or more other agents, which are also typically administered in the form of pharmaceutical compositions. Where desired, the crystalline forms of the invention and other agent(s) may be mixed into a preparation or both components may be formulated into separate preparations to use them in combination separately or at the same time.

In some embodiments, the concentration of the crystalline forms of Formula I in the pharmaceutical compositions of the present invention is less than 100%, 90%, 80%, 70%, 60%, 50%, 40%, 30%, 20%, 19%, 18%, 17%, 16%, 15%,14%, 13%, 12%, 11%, 10%, 9%, 8%, 7%, 6%, 5%, 4%, 3%, 2%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002%, or 0.0001% w/w, w/v or v/v.

In some embodiments, the concentration of the crystalline forms of Formula I in the pharmaceutical compositions of the present invention is greater than 90%, 80%, 70%, 60%, 50%, 40%, 30%, 20%, 19.75%, 19.50%, 19.25% 19%, 18.75%, 18.50%, 18.25% 18%, 17.75%, 17.50%, 17.25% 17%, 16.75%, 16.50%, 16.25% 16%, 15.75%, 15.50%, 15.25% 15%, 14.75%, 14.50%, 14.25% 14%, 13.75%, 13.50%, 13.25% 13%, 12.75%, 12.50%, 12.25% 12%, 11.75%, 11.50%, 11.25% 11%, 10.75%, 10.50%, 10.25% 10%, 9.75%, 9.50%, 9.25% 9%, 8.75%, 8.50%, 8.25% 8%, 7.75%, 7.50%, 7.25% 7%, 6.75%, 6.50%, 6.25% 6%, 5.75%, 5.50%, 5.25% 5%, 4.75%, 4.50%, 4.25%, 4%, 3.75%, 3.50%, 3.25%, 3%, 2.75%, 2.50%, 2.25%, 2%, 1.75%, 1.50%, 125%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002%, or 0.0001% w/w, w/v, or v/v.

In some embodiments, the concentration of a crystalline forms of Formula I in the pharmaceutical compositions of the present invention is in the range from approximately 0.0001% to approximately 50%, approximately 0.001% to approximately 40 %, approximately 0.01% to approximately 30%, approximately 0.02% to approximately 29%, approximately 0.03% to approximately 28%, approximately 0.04% to approximately 27%, approximately 0.05% to approximately 26%, approximately 0.06% to approximately 25%, approximately 0.07% to approximately 24%, approximately 0.08% to approximately 23%, approximately 0.09% to approximately 22%, approximately 0.1% to approximately 21%, approximately 0.2% to approximately 20%, approximately 0.3% to approximately 19%, approximately 0.4% to approximately 18%, approximately 0.5% to approximately 17%, approximately 0.6% to approximately 16%, approximately 0.7% to approximately 15%, approximately 0.8% to approximately 14%, approximately 0.9% to approximately 12%, approximately 1% to approximately 10% w/w, w/v or v/v.

In some embodiments, the amount of a crystalline form of Formula I in 1 mL of the pharmaceutical composition is equal to or less than 10 g, 9.5 g, 9.0 g, 8.5 g, 8.0 g, 7.5 g, 7.0 g, 6.5 g, 6.0 g, 5.5 g, 5.0 g, 4.5 g, 4.0 g, 3.5 g, 3.0 g, 2.5 g, 2.0 g, 1.5 g, 1.0 g, 0.95 g, 0.9 g, 0.85 g, 0.8 g, 0.75 g, 0.7 g, 0.65 g, 0.6 g, 0.55 g, 0.5 g, 0.45 g, 0.4 g, 0.35 g, 0.3 g, 0.25 g, 0.2 g, 0.15 g, 0.1 g, 0.09 g, 0.08 g, 0.07 g, 0.06 g, 0.05 g, 0.04 g, 0.03 g, 0.02 g, 0.01 g, 0.009 g, 0.008 g, 0.007 g, 0.006 g, 0.005 g, 0.004 g, 0.003 g, 0.002 g, 0.001 g, 0.0009 g, 0.0008 g, 0.0007 g, 0.0006 g, 0.0005 g, 0.0004 g, 0.0003 g, 0.0002 g, or 0.0001 g.

In some embodiments, the amount of a crystalline form of Formula I in 1 mL of the pharmaceutical composition is more than 0.0001 g, 0.0002 g, 0.0003 g, 0.0004 g, 0.0005 g, 0.0006 g, 0.0007 g, 0.0008 g, 0.0009 g, 0.001 g, 0.0015 g, 0.002 g, 0.0025 g, 0.003 g, 0.0035 g, 0.004 g, 0.0045 g, 0.005 g, 0.0055 g, 0.006 g, 0.0065 g, 0.007 g, 0.0075 g, 0.008 g, 0.0085 g, 0.009 g, 0.0095 g, 0.01 g, 0.015 g, 0.02 g, 0.025 g, 0.03 g, 0.035 g, 0.04 g, 0.045 g, 0.05 g, 0.055 g, 0.06 g, 0.065 g, 0.07 g, 0.075 g, 0.08 g, 0.085 g, 0.09 g, 0.095 g, 0.1 g, 0.15 g, 0.2 g, 0.25 g, 0.3 g, 0.35 g, 0.4 g, 0.45 g, 0.5 g, 0.55 g, 0.6 g, 0.65 g, 0.7 g, 0.75 g, 0.8 g, 0.85 g, 0.9 g, 0.95 g, 1 g, 1.5 g, 2 g, 2.5, 3 g, 3.5, 4 g, 4.5 g, 5 g, 5.5 g, 6 g, 6.5g, 7 g, 7.5g, 8 g, 8.5 g, 9 g, 9.5 g, or 10 g.

In some embodiments, the amount of a crystalline form of Formula I in 1 mL of the pharmaceutical composition is in the range of 0.0001-10 g, 0.0005-9 g, 0.001-8 g, 0.005-7 g, 0.01-6 g, 0.05-5 g, 0.1-4 g, 0.5-4 g, or 1-3 g.

The crystalline forms of Formula I according to the invention are effective over a wide dosage range. For example, in the treatment of adult humans, dosages from 0.01 to 1000 mg, from 0.5 to 100 mg, from 1 to 50 mg per day, and from 5 to 40 mg per day are examples of dosages that may be used. An exemplary dosage is 100 to 2000 mg per day. The exact dosage will depend upon the route of administration, the form in which the crystalline forms of Formula I are administered, the subject to be treated, the body weight of the subject to be treated, and the preference and experience of the attending physician.

Described below are non-limiting exemplary pharmaceutical compositions and methods for preparing the same.

### Pharmaceutical compositions for oral administration

In some embodiments, the invention provides a pharmaceutical composition for oral administration comprising the crystalline forms of Formula I of the present invention, and a pharmaceutical excipient suitable for oral administration.

In some embodiments, the invention provides a solid pharmaceutical composition for oral administration containing: (i) an effective amount of a crystalline form of Formula I of the present invention; optionally (ii) an effective amount of a second agent; and (iii) one or more pharmaceutical excipients suitable for oral administration. In some embodiments, the composition further contains: (iv) an effective amount of a third agent.

In some embodiments, the pharmaceutical composition may be a liquid pharmaceutical composition suitable for oral consumption. Pharmaceutical compositions of the invention suitable for oral administration can be presented as discrete dosage forms, such as capsules, cachets, or tablets, or liquids or aerosol sprays each containing a predetermined amount of an active ingredient as a powder or in granules, a solution, or a suspension in an aqueous or non-aqueous liquid, an oil-in-water emulsion, or a water-in-oil liquid emulsion. Such dosage forms can be prepared by any of the methods of pharmacy, but all methods include the step of bringing the active ingredient into association with the carrier, which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet can be prepared by compression or molding, optionally with one or more accessory ingredients. Compressed tablets can be prepared by compressing in a suitable machine the active ingredient in a free-flowing form such as powder or granules, optionally mixed with an excipient such as, but not limited to, a binder, a lubricant, an inert diluent, and/or a surface active or dispersing agent. Molded tablets can be made by molding in a suitable machine a mixture of the powdered compound moistened with an inert liquid diluent.

In some embodiments, the pharmaceutical compositions for oral delivery may be in the form of capsules, tablets, lozenges, aqueous or oily suspensions, granules, powders, emulsions, syrups, or elixirs. In some embodiments, the pharmaceutically acceptable vehicle is an oral capsule.

Orally administered compositions may contain one or more optional agents, for example, sweetening agents such as fructose, aspartame or saccharin, flavoring agents such as peppermint, oil of wintergreen or cherry coloring agents and preserving agents, to provide a pharmaceutically palatable preparation. Moreover, where in tablet or pill form, the pharmaceutical compositions may be coated to delay disintegration and absorption in the gastrointestinal tract, thereby providing a sustained action over an extended period of time. Selectively permeable membranes surrounding an osmotically active driving compound are also suitable for orally administering the compounds and compositions disclosed herein. In these later platforms, liquid from the environment surrounding the capsule is imbibed by the driving compound, which swells to displace the agent or agent composition through an aperture. These delivery platforms can provide an essentially zero order delivery profile as opposed to the spiked profiles of immediate release formulations. A time delay material such as glycerol monostearate or glycerol stearate may also be used. Oral compositions can include standard vehicles such as mannitol, lactose, starch, magnesium stearate, sodium saccharine, cellulose, magnesium carbonate, croscarmellose sodium, silicon dioxide, etc. Such vehicles are preferably of pharmaceutical grade. For oral liquid preparations such as, for example, suspensions, elixirs and solutions, suitable carriers, excipients or diluents include water, saline, alkylene glycols (e.g., propylene glycol), polyalkylene glycols (e. g., polyethylene glycol) oils, alcohols, slightly acidic buffers between pH 4 and pH 6 (e.g., acetate, citrate, ascorbate at between about 5 mM to about 50 mM), etc. Additionally, flavoring agents, preservatives, coloring agents, bile salts, acylcarnitines and the like may be added.

This invention further encompasses anhydrous pharmaceutical compositions and dosage forms comprising the crystalline forms of Formula I. Anhydrous pharmaceutical compositions and dosage forms of the invention can be prepared using anhydrous or low moisture containing ingredients and low moisture or low humidity conditions. Pharmaceutical compositions and dosage forms of the invention which contain lactose can be made anhydrous if substantial contact with moisture and/or humidity during manufacturing, packaging, and/or storage is expected. An anhydrous pharmaceutical composition may be prepared and stored such that its anhydrous nature is maintained. Accordingly, anhydrous compositions may be packaged using materials known to prevent exposure to water such that they can be included in suitable formulary kits. Examples of suitable packaging include, but are not limited to, hermetically sealed foils, plastic or the like, unit dose containers, blister packs, and strip packs.

The crystalline forms of Formula I can be combined in an intimate admixture with a pharmaceutical carrier according to conventional pharmaceutical compounding techniques. The carrier can take a wide variety of forms depending on the form of preparation desired for administration. In preparing the compositions for an oral dosage form, any of the usual pharmaceutical media can be employed as carriers, such as, for example, water, glycols, oils, alcohols, flavoring agents, preservatives, coloring agents, and the like in the case of oral liquid preparations (such as suspensions, solutions, and elixirs) or aerosols; or carriers such as starches, sugars, micro-crystalline cellulose, diluents, granulating agents, lubricants, binders, and disintegrating agents can be used in the case of oral solid preparations, in some embodiments without employing the use of lactose. For example, suitable carriers include powders, capsules, and tablets, with the solid oral preparations. If desired, tablets can be coated by standard aqueous or nonaqueous techniques.

Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, microcrystalline cellulose, and mixtures thereof.

Examples of suitable fillers for use in the pharmaceutical compositions and dosage forms disclosed herein include, but are not limited to, talc, calcium carbonate (e.g., granules or powder), microcrystalline cellulose, powdered cellulose, dextrates, kaolin, mannitol, silicic acid, sorbitol, starch, pre-gelatinized starch, and mixtures thereof.

Disintegrants may be used in the compositions of the invention to provide tablets that disintegrate when exposed to an aqueous environment. Too much of a disintegrant may produce tablets which may disintegrate in the bottle. Too little may be insufficient for disintegration to occur and may thus alter the rate and extent of release of the active ingredient(s) from the dosage form. Thus, a sufficient amount of disintegrant that is neither too little nor too much to detrimentally alter the release of the active ingredient(s) may be used to form the dosage forms of the polymorphs disclosed herein. The amount of disintegrant used may vary based upon the type of formulation and mode of administration, and may be readily discernible to those of ordinary skill in the art. About 0.5 to about 15 weight percent of disintegrant, or about 1 to about 5 weight percent of disintegrant, may be used in the pharmaceutical composition. Disintegrants that can be used to form pharmaceutical compositions and dosage forms of the invention include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums or mixtures thereof.

Lubricants which can be used to form pharmaceutical compositions and dosage forms of the invention include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethylaureate, agar, or mixtures thereof. Additional lubricants include, for example, a syloid silica gel, a coagulated aerosol of synthetic silica, or mixtures thereof. A lubricant can optionally be added, in an amount of less than about 1 weight percent of the pharmaceutical composition.

In some cases, colloid particles include at least one cationic agent and at least one non-ionic surfactant such as a poloxamer, tyloxapol, a polysorbate, a polyoxyethylene castor oil derivative, a sorbitan ester, or a polyoxyl stearate. In some cases, the cationic agent is an alkylamine, a tertiary alkyl amine, a quaternary ammonium compound, a cationic lipid, an amino alcohol, a biguanidine salt, a cationic compound or a mixture thereof. In some cases the cationic agent is a biguanidine salt such as chlorhexidine, polyaminopropyl biguanidine, phenformin, alkylbiguanidine, or a mixture thereof. In some cases, the quaternary ammonium compound is a benzalkonium halide, lauralkonium halide, cetrimide, hexadecyltrimethylammonium halide, tetradecyltrimethylammonium halide, dodecyltrimethylammonium halide, cetrimonium halide, benzethonium halide, behenalkonium halide, cetalkonium halide, cetethyldimonium halide, cetylpyridinium halide, benzododecinium halide, chlorallyl methenamine halide, myristylalkonium halide, stearalkonium halide or a mixture of two or more thereof. In some cases, cationic agent is a benzalkonium chloride, lauralkonium chloride, benzododecinium bromide, benzethenium chloride, hexadecyltrimethylammonium bromide, tetradecyltrimethylammonium bromide, dodecyltrimethylammonium bromide or a mixture of two or more thereof. In some cases, the oil phase is mineral oil and light mineral oil, medium chain triglycerides (MCT), coconut oil; hydrogenated oils comprising hydrogenated cottonseed oil, hydrogenated palm oil, hydrogenate castor oil or hydrogenated soybean oil; polyoxyethylene hydrogenated castor oil derivatives comprising poluoxyl-40 hydrogenated castor oil, polyoxyl-60 hydrogenated castor oil or polyoxyl-100 hydrogenated castor oil.

When aqueous suspensions and/or elixirs are desired for oral administration, the active ingredient therein may be combined with various sweetening or flavoring agents, coloring matter or dyes and, if so desired, emulsifying and/or suspending agents, together with such diluents as water, ethanol, propylene glycol, glycerin and various combinations thereof.

The tablets can be uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Surfactant which can be used to form pharmaceutical compositions and dosage forms of the invention include, but are not limited to, hydrophilic surfactants, lipophilic surfactants, and mixtures thereof. That is, a mixture of hydrophilic surfactants may be employed, a mixture of lipophilic surfactants may be employed, or a mixture of at least one hydrophilic surfactant and at least one lipophilic surfactant may be employed.

A suitable hydrophilic surfactant may generally have an HLB value of at least 10, while suitable lipophilic surfactants may generally have an HLB value of or less than about 10. An empirical parameter used to characterize the relative hydrophilicity and hydrophobicity of non-ionic amphiphilic compounds is the hydrophilic-lipophilic balance ("HLB" value). Surfactants with lower HLB values are more lipophilic or hydrophobic, and have greater solubility in oils, while surfactants with higher HLB values are more hydrophilic, and have greater solubility in aqueous solutions. Hydrophilic surfactants are generally considered to be those compounds having an HLB value greater than about 10, as well as anionic, cationic, or zwitterionic compounds for which the HLB scale is not generally applicable. Similarly, lipophilic (i.e., hydrophobic) surfactants are compounds having an HLB value equal to or less than about 10. However, HLB value of a surfactant is merely a rough guide generally used to enable formulation of industrial, pharmaceutical and cosmetic emulsions.

Hydrophilic surfactants may be either ionic or non-ionic. Suitable ionic surfactants include, but are not limited to, alkylammonium salts; fusidic acid salts; fatty acid derivatives of amino acids, oligopeptides, and polypeptides; glyceride derivatives of amino acids, oligopeptides, and polypeptides; lecithins and hydrogenated lecithins; lysolecithins and hydrogenated lysolecithins; phospholipids and derivatives thereof; lysophospholipids and derivatives thereof; carnitine fatty acid ester salts; salts of alkylsulfates; fatty acid salts; sodium docusate; acylactylates; mono- and di-acetylated tartaric acid esters of mono- and di-glycerides; succinylated mono- and di-glycerides; citric acid esters of mono- and di-glycerides; and mixtures thereof.

Within the aforementioned group, ionic surfactants include, by way of example: lecithins, lysolecithin, phospholipids, lysophospholipids and derivatives thereof; carnitine fatty acid ester salts; salts of alkylsulfates; fatty acid salts; sodium docusate; acylactylates; mono- and di-acetylated tartaric acid esters of mono- and di-glycerides; succinylated mono- and di-glycerides; citric acid esters of mono- and di-glycerides; and mixtures thereof.

Ionic surfactants may be the ionized forms of lecithin, lysolecithin, phosphatidylcholine, phosphatidylethanolamine, phosphatidylglycerol, phosphatidic acid, phosphatidylserine, lysophosphatidylcholine, lysophosphatidylethanolamine, lysophosphatidylglycerol, lysophosphatidic acid, lysophosphatidylserine, PEG-phosphatidylethanolamine, PVP-phosphatidylethanolamine, lactylic esters of fatty acids, stearoyl-2-lactylate, stearoyl lactylate, succinylated monoglycerides, mono/diacetylated tartaric acid esters of mono/diglycerides, citric acid esters of mono/diglycerides, cholylsarcosine, caproate, caprylate, caprate, laurate, myristate, palmitate, oleate, ricinoleate, linoleate, linolenate, stearate, lauryl sulfate, teracecyl sulfate, docusate, lauroyl carnitines, palmitoyl carnitines, myristoyl carnitines, and salts and mixtures thereof.

Hydrophilic non-ionic surfactants may include, but are not limited to, alkylglucosides; alkylmaltosides; alkylthioglucosides; lauryl macrogolglycerides; polyoxyalkylene alkyl ethers such as polyethylene glycol alkyl ethers; polyoxyalkylene alkylphenols such as polyethylene glycol alkyl phenols; polyoxyalkylene alkyl phenol fatty acid esters such as polyethylene glycol fatty acids monoesters and polyethylene glycol fatty acids diesters; polyethylene glycol glycerol fatty acid esters; polyglycerol fatty acid esters; polyoxyalkylene sorbitan fatty acid esters such as polyethylene glycol sorbitan fatty acid esters; hydrophilic transesterification products of a polyol with at least one member of the group consisting of glycerides, vegetable oils, hydrogenated vegetable oils, fatty acids, and sterols; polyoxyethylene sterols, derivatives, and analogues thereof; polyoxyethylated vitamins and derivatives thereof; polyoxyethylene-polyoxypropylene block copolymers; and mixtures thereof; polyethylene glycol sorbitan fatty acid esters and hydrophilic transesterification products of a polyol with at least one member of the group consisting of triglycerides, vegetable oils, and hydrogenated vegetable oils. The polyol may be glycerol, ethylene glycol, polyethylene glycol, sorbitol, propylene glycol, pentaerythritol, or a saccharide.

Other hydrophilic-non-ionic surfactants include, without limitation, PEG-10 laurate, PEG-12 laurate, PEG-20 laurate, PEG-32 laurate, PEG-32 dilaurate, PEG-12 oleate, PEG-15 oleate, PEG-20 oleate, PEG-20 dioleate, PEG-32 oleate, PEG-200 oleate, PEG-400 oleate, PEG-15 stearate, PEG-32 distearate, PEG-40 stearate, PEG-100 stearate, PEG-20 dilaurate, PEG-25 glyceryl trioleate, PEG-32 dioleate, PEG-20 glyceryl laurate, PEG-30 glyceryl laurate, PEG-20 glyceryl stearate, PEG-20 glyceryl oleate, PEG-30 glyceryl oleate, PEG-30 glyceryl laurate, PEG-40 glyceryl laurate, PEG-40 palm kernel oil, PEG-50 hydrogenated castor oil, PEG-40 castor oil, PEG-35 castor oil, PEG-60 castor oil, PEG-40 hydrogenated castor oil, PEG-60 hydrogenated castor oil, PEG-60 corn oil, PEG-6 caprate/caprylate glycerides, PEG-8 caprate/caprylate glycerides, polyglyceryl-10 laurate, PEG-30 cholesterol, PEG-25 phyto sterol, PEG-30 soya sterol, PEG-20 trioleate, PEG-40 sorbitan oleate, PEG-80 sorbitan laurate, polysorbate 20, polysorbate 80, POE-9 lauryl ether, POE-23 lauryl ether, POE-10 oleyl ether, POE-20 oleyl ether, POE-20 stearyl ether, tocopheryl PEG-100 succinate, PEG-24 cholesterol, polyglyceryl-10oleate, Tween 40, Tween 60, sucrose monostearate, sucrose monolaurate, sucrose monopalmitate, PEG 10-100 nonyl phenol series, PEG 15-100 octyl phenol series, and poloxamers.

Suitable lipophilic surfactants include, by way of example only: fatty alcohols; glycerol fatty acid esters; acetylated glycerol fatty acid esters; lower alcohol fatty acids esters; propylene glycol fatty acid esters; sorbitan fatty acid esters; polyethylene glycol sorbitan fatty acid esters; sterols and sterol derivatives; polyoxyethylated sterols and sterol derivatives; polyethylene glycol alkyl ethers; sugar esters; sugar ethers; lactic acid derivatives of mono- and di-glycerides; hydrophobic transesterification products of a polyol with at least one member of the group consisting of glycerides, vegetable oils, hydrogenated vegetable oils, fatty acids and sterols; oil-soluble vitamins/vitamin derivatives; and mixtures thereof. Within this group, preferred lipophilic surfactants include glycerol fatty acid esters, propylene glycol fatty acid esters, and mixtures thereof, or are hydrophobic transesterification products of a polyol with at least one member of the group consisting of vegetable oils, hydrogenated vegetable oils, and triglycerides.

In one embodiment, the composition may include a solubilizer to ensure good solubilization and/or dissolution of the compound of the present invention and to minimize precipitation of the compound of the present invention. This can be especially important for compositions for non-oral use, e.g., compositions for injection. A solubilizer may also be added to increase the solubility of the hydrophilic drug and/or other components, such as surfactants, or to maintain the composition as a stable or homogeneous solution or dispersion.

Examples of suitable solubilizers include, but are not limited to, the following: alcohols and polyols, such as ethanol, isopropyl alcohol, butanol, benzyl alcohol, ethylene glycol, propylene glycol, butanediols and isomers thereof, glycerol, pentaerythritol, sorbitol, mannitol, transcutol, dimethyl isosorbide, polyethylene glycol, polypropylene glycol, polyvinylalcohol, hydroxypropyl methylcellulose and other cellulose derivatives, cyclodextrins and cyclodextrin derivatives; ethers of polyethylene glycols having an average molecular weight of about 200 to about 6000, such as tetrahydrofurfuryl alcohol PEG ether (glycofurol) or methoxy PEG; amides and other nitrogen-containing compounds such as 2-pyrrolidone, 2-piperidone, ε-caprolactam, N-alkylpyrrolidone, N-hydroxyalkylpyrrolidone, N-alkylpiperidone, N-alkylcaprolactam, dimethylacetamide and polyvinylpyrrolidone; esters such as ethyl propionate, tributylcitrate, acetyl triethylcitrate, acetyl tributyl citrate, triethylcitrate, ethyl oleate, ethyl caprylate, ethyl butyrate, triacetin, propylene glycol monoacetate, propylene glycol diacetate, ε-caprolactone and isomers thereof, δ-valerolactone and isomers thereof, β-butyrolactone and isomers thereof; and other solubilizers known in the art, such as dimethyl acetamide, dimethyl isosorbide, N-methyl pyrrolidones, monooctanoin, diethylene glycol monoethyl ether, and water. In various embodiments, a solubilizer comprising polyglycol mono-and di-esters of 12-hydroxystearic acid and about 30% free polyethylene glycol (available as Solutol HS 15) is used as a solubilizer.

Mixtures of solubilizers may also be used. Examples include, but not limited to, triacetin, triethylcitrate, ethyl oleate, ethyl caprylate, dimethylacetamide, N-methylpyrrolidone, N-hydroxyethylpyrrolidone, polyvinylpyrrolidone, hydroxypropyl methylcellulose, hydroxypropyl cyclodextrins, ethanol, polyethylene glycol 200-100, glycofurol, transcutol, propylene glycol, and dimethyl isosorbide. Particularly preferred solubilizers include sorbitol, glycerol, triacetin, ethyl alcohol, PEG-400, glycofurol and propylene glycol.

The amount of solubilizer that can be included is not particularly limited. The amount of a given solubilizer may be limited to a bioacceptable amount, which may be readily determined by one of skill in the art. In some circumstances, it may be advantageous to include amounts of solubilizers far in excess of bioacceptable amounts, for example to maximize the concentration of the drug, with excess solubilizer removed prior to providing the composition to a subject using conventional techniques, such as distillation or evaporation. Thus, if present, the solubilizer can be in a weight ratio of 10%, 25%, 50%, 100%, or up to about 200% by weight, based on the combined weight of the drug, and other excipients. If desired, very small amounts of solubilizer may also be used, such as 5%, 2%, 1%, or even less. Typically, the solubilizer may be present in an amount of about 1% to about 100%, more typically about 5% to about 25% by weight.

The composition can further include one or more pharmaceutically acceptable additives and excipients. Such additives and excipients include, without limitation, detackifiers, anti-foaming agents, buffering agents, polymers, antioxidants, preservatives, chelating agents, viscomodulators, tonicifiers, flavorants, colorants, odorants, opacifiers, suspending agents, binders, fillers, plasticizers, lubricants, and mixtures thereof.

In addition, an acid or a base may be incorporated into the composition to facilitate processing, to enhance stability, or for other reasons. Examples of pharmaceutically acceptable bases include amino acids, amino acid esters, ammonium hydroxide, potassium hydroxide, sodium hydroxide, sodium hydrogen carbonate, aluminum hydroxide, calcium carbonate, magnesium hydroxide, magnesium aluminum silicate, synthetic aluminum silicate, synthetic hydrocalcite, magnesium aluminum hydroxide, diisopropylethylamine, ethanolamine, ethylenediamine, triethanolamine, triethylamine, triisopropanolamine, trimethylamine, tris(hydroxymethyl)aminomethane (TRIS) and the like. Also suitable are bases that are salts of a pharmaceutically acceptable acid, such as acetic acid, acrylic acid, adipic acid, alginic acid, alkanesulfonic acid, amino acids, ascorbic acid, benzoic acid, boric acid, butyric acid, carbonic acid, citric acid, fatty acids, formic acid, fumaric acid, gluconic acid, hydroquinosulfonic acid, isoascorbic acid, lactic acid, maleic acid, oxalic acid, para-bromophenylsulfonic acid, propionic acid, p-toluenesulfonic acid, salicylic acid, stearic acid, succinic acid, tannic acid, tartaric acid, thioglycolic acid, toluene sulfonic acid, uric acid, and the like. Salts of polyprotic acids, such as sodium phosphate, disodium hydrogen phosphate, and sodium dihydrogen phosphate can also be used. When the base is a salt, the cation can be any convenient and pharmaceutically acceptable cation, such as ammonium, alkali metals, alkaline earth metals, and the like. Example may include, but not limited to, sodium, potassium, lithium, magnesium, calcium and ammonium.

Suitable acids are pharmaceutically acceptable organic or inorganic acids. Examples of suitable inorganic acids include hydrochloric acid, hydrobromic acid, hydriodic acid, sulfuric acid, nitric acid, boric acid, phosphoric acid, and the like. Examples of suitable organic acids include acetic acid, acrylic acid, adipic acid, alginic acid, alkanesulfonic acids, amino acids, ascorbic acid, benzoic acid, boric acid, butyric acid, carbonic acid, citric acid, fatty acids, formic acid, fumaric acid, gluconic acid, hydroquinosulfonic acid, isoascorbic acid, lactic acid, maleic acid, methane sulfonic acid, oxalic acid, para-bromophenylsulfonic acid, propionic acid, p-toluenesulfonic acid, salicylic acid, stearic acid, succinic acid, tannic acid, tartaric acid, thioglycolic acid, toluenesulfonic acid, uric acid and the like.

### Pharmaceutical compositions for injection

In some embodiments, the invention provides a pharmaceutical composition for injection containing a compound of the present invention and a pharmaceutical excipient suitable for injection. Components and amounts of agents in the compositions are as described herein.

The forms in which the novel compositions of the present invention may be incorporated for administration by injection include aqueous or oil suspensions, or emulsions, with sesame oil, corn oil, cottonseed oil, or peanut oil, as well as elixirs, mannitol, dextrose, or a sterile aqueous solution, and similar pharmaceutical vehicles.

Aqueous solutions in saline are also conventionally used for injection. Ethanol, glycerol, propylene glycol, liquid polyethylene glycol, and the like (and suitable mixtures thereof), cyclodextrin derivatives, and vegetable oils may also be employed. The proper fluidity can be maintained, for example, by the use of a coating, such as lecithin, for the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like.

Sterile injectable solutions are prepared by incorporating the compound of the present invention in the required amount in the appropriate solvent with various other ingredients as enumerated above, as required, followed by sterile filtration. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, certain desirable methods of preparation are vacuum-drying and freeze-drying techniques which yield a powder of the active ingredient plus any additional desired ingredient from a previously sterile-filtered solution thereof.

In some embodiments, the crystalline forms of Formula I are formulated as liquid formulations for intravenous administration. Water is a preferred vehicle when the crystalline forms of Formula I are administered intravenously. Saline solutions and aqueous dextrose and glycerol solutions can also be employed as liquid vehicles, particularly for inject able solutions. Suitable pharmaceutical vehicles also include excipients such as starch, cellulose, glucose, lactose, sucrose, gelatin, malt, rice, chalk, silica gel, sodium stearate, glycerol monostearate, talc, sodium chloride, dried skim milk, glycerol, propylene, glycol, water, ethanol, etc. The present compositions, if desired, can also contain minor amounts of wetting or emulsifying agents or pH buffering agents. In addition, auxiliary, stabilizing, thickening, lubricating and coloring agents may be used.

Pharmaceutical compositions comprising the crystalline forms of Formula I may be manufactured by means of conventional mixing, dissolving, granulating, dragee-making, levigating, emulsifying, encapsulating, entrap ping or lyophilizing processes. Pharmaceutical compositions may be formulated in conventional manner using one or more physiologically acceptable carriers, diluents, excipients or auxiliaries, which facilitate processing of crystalline forms of Formula I into preparations which can be used pharmaceutically. Proper formulation is dependent upon the route of administration chosen. The present pharmaceutical compositions can take the form of solutions, suspensions, emulsion, tablets, pills, pellets, capsules, capsules containing liquids, powders, sustained-release formulations, suppositories, emulsions, aerosols, sprays, suspensions, or any other form suitable for use.

### Pharmaceutical compositions for topical (e.g., transdermal) delivery

In some embodiments, the invention provides a pharmaceutical composition for transdermal delivery containing a compound of the present invention and at least one pharmaceutical excipient suitable for transdermal delivery.

Compositions of the present invention can be formulated into preparations in solid, semi-solid, or liquid forms suitable for local or topical administration, such as gels, water soluble jellies, creams, lotions, suspensions, foams, powders, slurries, ointments, solutions, oils, pastes, suppositories, sprays, emulsions, saline solutions, dimethylsulfoxide (DMSO)-based solutions. In general, carriers with higher densities are capable of providing an area with a prolonged exposure to the active ingredients. In contrast, a solution formulation may provide more immediate exposure of the active ingredient to the chosen area.

The pharmaceutical compositions also may comprise suitable solid or gel phase carriers or excipients, which are compounds that allow increased penetration of, or assist in the delivery of, therapeutic molecules across the stratum corneum permeability barrier of the skin. There are many of these penetration-enhancing molecules known to those trained in the art of topical formulation. Examples of such carriers and excipients include, but are not limited to, humectants (e.g., urea), glycols (e.g., propylene glycol), alcohols (e.g., ethanol), fatty acids (e.g., oleic acid), surfactants (e.g., isopropyl myristate and sodium lauryl sulfate), pyrrolidones, glycerol monolaurate, sulfoxides, terpenes (e.g., menthol), amines, amides, alkanes, alkanols, water, calcium carbonate, calcium phosphate, various sugars, starches, cellulose derivatives, gelatin, and polymers such as polyethylene glycols.

Another exemplary formulation for use in the methods of the present invention employs transdermal delivery devices ("patches"). Such transdermal patches may be used to provide continuous or discontinuous infusion of a compound of the present invention in controlled amounts, either with or without another agent.

The construction and use of transdermal patches for the delivery of pharmaceutical agents is known in the art. See, *e.g*., U.S. Pat. Nos. 5,023,252, 4,992,445 and 5,001,139. Such patches may be constructed for continuous, pulsatile, or on demand delivery of pharmaceutical agents.

### Pharmaceutical compositions for inhalation.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described supra. Preferably the compositions are administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices that deliver the formulation in an appropriate manner.

Liquid drug formulations suitable for use with nebulizers and liquid spray devices and EHD aerosol devices will typically include the crystalline forms of Formula I with a pharmaceutically acceptable vehicle. Preferably, the pharmaceutically acceptable vehicle is a liquid such as alcohol, water, polyethylene glycol or perfluorocarbon. Optionally, another material may be added to alter the aerosol properties of the solution or suspension of the compounds disclosed herein. Preferably, this material is liquid such as an alcohol, glycol, polyglycol or a fatty acid. Other methods of formulating liquid drug solutions or suspension suitable for use in aerosol devices are known to those of skill in the art (see, e.g. Biesalski, US. Pat. No. 5,556,611).

### Pharmaceutical composition for treating ophthalmic disorders

The composition is formulated for ocular administration and it contains an effective amount of one or more crystalline forms of the present invention and a pharmaceutical excipient suitable for ocular administration. Pharmaceutical compositions of the invention suitable for ocular administration can be presented as discrete dosage forms, such as drops or sprays each containing a predetermined amount of an active ingredient in a solution, or a suspension in an aqueous or non-aqueous liquid, an oil-in-water emulsion, or a water-in-oil liquid emulsion. Eye drops may be prepared by dissolving the active ingredient in a sterile aqueous solution such as physiological saline, buffering solution, etc., or by combining powder compositions to be dissolved before use. Other vehicles may be chosen, as is known in the art, including but not limited to: balance salt solution, saline solution, water soluble polyethers such as polyethyene glycol, polyvinyls, such as polyvinyl alcohol and povidone, cellulose derivatives such as methylcellulose and hydroxypropyl methylcellulose, petroleum derivatives such as mineral oil and white petrolatum, animal fats such as lanolin, polymers of acrylic acid such as carboxypolymethylene gel, vegetable fats such as peanut oil and polysaccharides such as dextrans, and glycosaminoglycans such as sodium hyaluronate. If desired, additives ordinarily used in the eye drops can be added. Such additives include isotonizing agents (e.g., sodium chloride, etc.), buffer agent (e.g., boric acid, sodium monohydrogen phosphate, sodium dihydrogen phosphate, etc.), preservatives (e.g., benzalkonium chloride, benzethonium chloride, chlorobutanol, etc.), thickeners (e.g., saccharide such as lactose, mannitol, maltose, etc.; e.g., hyaluronic acid or its salt such as sodium hyaluronate, potassium hyaluronate, etc.; e.g., mucopolysaccharide such as chondroitin sulfate, etc.; e.g., sodium polyacrylate, carboxyvinyl polymer, crosslinked polyacrylate, polyvinyl alcohol, polyvinyl pyrrolidone, methyl cellulose, hydroxy propyl methylcellulose, hydroxyethyl cellulose, carboxymethyl cellulose, hydroxy propyl cellulose or other agents known to those skilled in the art).

### Other pharmaceutical compositions

Pharmaceutical compositions may also be prepared from compositions described herein and one or more pharmaceutically acceptable excipients suitable for sublingual, buccal, rectal, intraosseous, intraocular, intranasal, epidural, or intraspinal administration. Preparations for such pharmaceutical compositions are well-known in the art. *See, e.g*., Anderson, Philip O.; Knoben, James E.; Troutman, William G, eds., Handbook of Clinical Drug Data, Tenth Edition, McGraw-Hill, 2002; Pratt and Taylor, eds., Principles of Drug Action, Third Edition, Churchill Livingston, New York, 1990; Katzung, ed., Basic and Clinical Pharmacology, Ninth Edition, McGraw Hill, 20037ybg; Goodman and Gilman, eds., The Pharmacological Basis of Therapeutics, Tenth Edition, McGraw Hill, 2001; Remingtons Pharmaceutical Sciences, 20th Ed., Lippincott Williams & Wilkins., 2000; Martindale, The Extra Pharmacopoeia, Thirty-Second Edition (The Pharmaceutical Press, London, 1999); all of which are incorporated by reference herein in their entirety.

In some embodiments, the crystalline forms of Formula I are formulated in rectal or vaginal compositions such as suppositories or retention enemas, e. g., containing conventional suppository bases such as cocoa butter or other glycerides.

In some embodiments, the crystalline forms of Formula I may also be formulated as a depot preparation. Such long acting formulations may be administered by implantation (for example subcutaneously or intra muscularly) or by intramuscular injection. Thus, for example, a crystalline form of Formula I may be formulated with suitable polymeric or hydrophobic materials (for example, as an emulsion in an acceptable oil) or ion exchange resins, or as sparingly soluble derivatives, for example, as a sparingly soluble salt.

In some embodiments, a crystalline form of Formula I is formulated as a pure active agent.

In some embodiments, a crystalline form of Formula I is formulated as a mixture with other crystalline forms of Formula I.

### Modes of Administration

Administration of the crystalline forms of the compound of Formula I, or the pharmaceutical composition of the invention can be effected by any method that enables delivery of the crystalline forms to the site of action. These methods include, but are not limited to, oral routes, intraduodenal routes, parenteral injection (including intravenous, intraarterial, subcutaneous, intramuscular, intravascular, intraperitoneal or infusion), topical (e.g. transdermal application), rectal administration, via local delivery by catheter or stent or through inhalation. The crystalline forms of the compound of Formula I, or the pharmaceutical composition of the invention can also be administered intraadiposally or intrathecally.

In some embodiments, the crystalline forms of the compound of Formula I and/or the pharmaceutical compositions thereof, are administered orally. In some embodiments, the crystalline forms of the compound of Formula I and/or the pharmaceutical compositions thereof, are administered for example, by infusion, bolus injection, topical administration through skin, absorption through epithelial or mucocutaneous linings (e.g., oral mucosa, rectal and intestinal mucosa, etc.).

The administration of the crystalline forms of the compound of Formula I can be systemic or local. Various delivery systems are known, (e.g., encapsulation in liposomes, microparticles, microcapsules, capsules, etc.) that can be used to administer the crystalline forms Formula I and/or pharmaceutical compositions thereof. Methods of administration include, but are not limited to, intradermal, intramuscular, intraperitoneal, intravenous, subcutaneous, intranasal, epidural, oral, sublingual, intranasal, intracerebral, intravaginal, transdermal, rectally, by inhalation, or topically, particularly to the ears, nose, eyes or skin. In some embodiments, the crystalline forms of the compound of Formula I and/or pharmaceutical compositions thereof are administered orally, for e.g. in form of an oral capsule.

In some embodiments, the crystalline forms of the compound of Formula I are administered by controlled/sustained release systems. The crystalline forms of the invention may be administered, for example, by local delivery from the struts of a stent, from a stent graft, from grafts, or from the cover or sheath of a stent. In some embodiments, a crystalline form of the invention is admixed with a matrix. Such a matrix may be a polymeric matrix, and may serve to bond the compound to the stent. Polymeric matrices suitable for such use, include, for example, lactone-based polyesters or copolyesters such as polylactide, polycaprolactonglycolide, polyorthoesters, polyanhydrides, polyaminoacids, polysaccharides, polyphosphazenes, poly (ether-ester) copolymers (e.g. PEO-PLLA); polydimethylsiloxane, poly(ethylenevinylacetate), acrylate-based polymers or copolymers (e.g. polyhydroxyethyl methylmethacrylate, polyvinyl pyrrolidinone), fluorinated polymers such as polytetrafluoroethylene and cellulose esters.

Suitable matrices may be nondegrading or may degrade with time, releasing the compound or compounds. Additional suitable polymeric materials that can be used with the crystalline forms of the invention are described for example in Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Langer et al., 1983, J. Macromol. Sci. Rev. Macromol. Chem. 23161; Levy et al., 1985, Science 228: 190; During et al., 1989, Ann. Neurol. 251351; and Howard et al., 1989, J. Neurosurg. 711105, each of which is incorporated herein by reference in its entirety.

In some embodiments, the crystalline forms of the compound of Formula I are administered via oral sustained release delivery systems. Polymers that can be used for oral sustained release include, for example, sodium carboxymethylcellulose, hydroxypropylcellulose, hydroxypropylmethylcellulose and hydroxyethylcellulose (e.g. hydroxypropylmethyl cellulose). Any suitable cellulose ethers can also be used for oral sustained release delivery systems.

In still other embodiments, enteric-coated preparations can be used for oral sustained release administration. Coating materials include, for example, polymers with a pH-dependent solubility (i.e., pH-controlled release), polymers with a slow or pH-dependent rate of swelling, dissolution or erosion (i.e., time-controlled release), polymers that are degraded by enzymes (i.e., enzyme-controlled release) and polymers that form firm layers that are destroyed by an increase in pressure (i.e., pressure-controlled release).

In still other embodiments, osmotic delivery systems are used for oral sustained release administration (Verma et al., Drug Dev. Ind. Pharm. 2000, 261695-708). In some embodiments, OROS^{™} osmotic devices are used for oral sustained release delivery devices, for example as disclosed in U.S. Pat. No. 3,845, 770 and U.S. Pat. No. 3,916,899.

In yet other embodiments, waxes can be used for oral sustained release administration. Examples of suitable sustained releasing waxes are disclosed in U.S. Pat. No. 3,402,240 (carnauba wax, candedilla wax, esparto wax and ouricury wax); U.S. Pat. No. 4,820,523 (hydrogenated vegetable oil, bees wax, caranuba wax, paraffin, candelillia, ozokerite and mixtures thereof); and U.S. Pat. No. 4,421,736 (mixture of paraffin and castor wax).

In some embodiments, drug-releasing lipid matrices can be used for oral sustained release administration. For example, solid microparticles of compositions and/or compounds disclosed herein may be coated with a thin controlled release layer of a lipid (e.g., glyceryl behenate and/or glyceryl palmitostearate) as disclosed in U.S. Pat. No. 6,375,987 and U.S. Pat. No. 6,379,700. The lipid-coated particles can optionally be compressed to form a tablet. Another controlled release lipid-based matrix material which is suitable for sustained release oral administration comprises polyglycolized glycerides as disclosed in U.S. Pat. No. 6,171,615.

In some embodiments, the different controlled-release systems described herein can be placed in proximity of the target of the crystalline form of Formula I and/or pharmaceutical compositions thereof, thus requiring only a fraction of the systemic dose (e.g., Goodson, in "Medical Applications of Controlled Release," supra, vol. 2, pp. 115-138 (1984)). In some embodiments, the amount of the crystalline forms of Formula I required is about 1/20, 1/15, 1/10, 1/9. 1/8. 1/7, 1/6, 1/5, 1/4, 1/3, or 1/2 of the systemic dose. Other controlled-release systems discussed in Langer, 1990, Science 24911527-1533 may also be used.

In still other embodiments, topical delivery systems are used for the delivery of the crystalline forms of the compounds of Formula I and/or pharmaceutical compositions thereof. For e.g. the systems described in LeBon, B., et al, Journal of Pain and Symptom Management, 2009, 37(5), 913 and Singh, V., et al., Asian Journal of Pharmaceutics, 2013, January-March, pp. 1-7 can be used.

The crystalline forms of the compound of Formula I and/ or pharmaceutical compositions thereof preferably, provide naproxen and pregabalin upon *in vivo* administration to a patient. While not wishing to bound by theory, the linker of the drug conjugate of crystalline forms of the compound of Formula I and/or pharmaceutical compositions thereof may be cleaved either chemically and/or enzymatically. One or more enzymes present in the stomach, intestinal lumen, intestinal tissue, blood, liver, brain and/or any other suitable tissue of a mammal may cleave the linker of crystalline forms of the compound of Formula I and/or pharmaceutical compositions thereof. The mechanism of cleavage is not important. While not wishing to bound by theory, the linker of crystalline forms the compound of Formula I and/ or pharmaceutical compositions thereof may be cleaved prior to absorption by the gastrointestinal tract (e.g., within the stomach or intestinal lumen) and/or after absorption by the gastrointestinal tract (e.g., in intestinal tissue, blood, liver or other suitable tissue of a mammal). In some embodiments, the linker of crystalline forms of the compound of Formula I and/or pharmaceutical compositions thereof is cleaved prior to absorption by the gastrointestinal tract, and both naproxen and pregabalin are absorbed into the systemic circulation conventionally. In some embodiments, the linker of crystalline forms of the compound of Formula I and/or pharmaceutical compositions thereof is cleaved after absorption by the gastrointestinal tract, and naproxen and pregabalin are absorbed into the systemic circulation either by passive diffusion, active transport, or both.

If the linker of crystalline forms of the compound of Formula I and/or pharmaceutical compositions thereof is cleaved after absorption by the gastrointestinal tract, naproxen and pregabalin may have the opportunity to be absorbed into the systemic circulation from the large intestine. In this situation, crystalline forms of the compound of Formula I and/ or pharmaceutical compositions thereof are preferably administered as sustained release systems. In some embodiments, crystalline forms of compound of Formula I and/or pharmaceutical compositions thereof are delivered by oral sustained release administration. In these embodiments, crystalline forms of compound of Formula I and/or pharmaceutical compositions thereof are administered once or twice per day, for example once per day.

### Dosages

The crystalline forms of Formula I and/or pharmaceutical compositions thereof, will generally be used in an amount effective to achieve the intended purpose. For use to treat or prevent diseases or disorders such as pain (e.g. neuropathic pain and musculoskeletal pain) and inflammatory disease (e.g., arthritis), the crystalline forms of Formula I and/or pharmaceutical compositions thereof are administered or applied in a therapeutically effective amount. The amount of the crystalline forms of Formula I and/or pharmaceutical compositions thereof that will be effective in the treatment of a particular disorder or condition disclosed herein will depend on the nature of the disorder or condition, and can be determined by standard clinical techniques known in the art as previously described. In addition, *in vitro* or *in vivo* assays may optionally be employed to help identify optimal dosage ranges.

The amount of a crystalline forms of Formula I and/or pharmaceutical compositions thereof administered will, of course, be dependent on, among other factors, the subject being treated, the weight of the subject, the severity of the affliction, the manner of administration and the judgment of the prescribing physician. For example, the dosage may be delivered in a pharmaceutical composition by a single administration, by multiple applications or controlled release. In some embodiments, the crystalline forms of Formula I and/or pharmaceutical compositions thereof are delivered by oral sustained release administration. In these embodiments, the crystalline forms of Formula I and/or pharmaceutical compositions thereof are administered five, four, three, two or one time per day. In some embodiments, the crystalline forms of Formula I and/or pharmaceutical compositions thereof are delivered by oral sustained release administration twice per day. In some embodiments, the crystalline forms of Formula I and/or pharmaceutical compositions thereof are delivered by oral sustained release administration once per day.

Dosing may be repeated intermittently, may be provided alone or in combination with other drugs and may continue as long as required for effective treatment of the disease state or disorder. In some embodiments, the dose of the crystalline forms of Formula I and/or pharmaceutical compositions thereof may be adjusted to provide between about 0.5 mg/day to about 20,000 mg/day of the drug conjugate. In some embodiments, the dose of the crystalline forms of Formula I and/or pharmaceutical compositions thereof may be adjusted to provide at least about 0.5 mg/day of the drug conjugate. In some embodiments, the dose of the crystalline forms of Formula I and/or pharmaceutical compositions thereof may be adjusted to provide at most about 20,000 mg/day of the drug conjugate.

In some embodiments, the dose of the crystalline forms of Formula I and/or pharmaceutical compositions thereof may be adjusted to provide between about 0.5 mg/day to about 5 mg/day of the drug conjugate, about 0.5 mg/day to about 50 mg/day of the drug conjugate, about 0.5 mg/day to about 100 mg/day of the drug conjugate, about 0.5 mg/day to about 200 mg/day of the drug conjugate, about 0.5 mg/day to about 300 mg/day of the drug conjugate, about 0.5 mg/day to about 500 mg/day of the drug conjugate, about 0.5 mg/day to about 1,000 mg/day of the drug conjugate, about 0.5 mg/day to about 2,000 mg/day of the drug conjugate, about 0.5 mg/day to about 5,000 mg/day of the drug conjugate, about 0.5 mg/day to about 10,000 mg/day of the drug conjugate, about 0.5 mg/day to about 20,000 mg/day of the drug conjugate, about 5 mg/day to about 50 mg/day of the drug conjugate, about 5 mg/day to about 100 mg/day of the drug conjugate, about 5 mg/day to about 200 mg/day of the drug conjugate, about 5 mg/day to about 300 mg/day of the drug conjugate, about 5 mg/day to about 500 mg/day of the drug conjugate, about 5 mg/day to about 1,000 mg/day of the drug conjugate, about 5 mg/day to about 2,000 mg/day of the drug conjugate, about 5 mg/day to about 5,000 mg/day of the drug conjugate, about 5 mg/day to about 10,000 mg/day of the drug conjugate, about 5 mg/day to about 20,000 mg/day of the drug conjugate, about 50 mg/day to about 100 mg/day of the drug conjugate, about 50 mg/day to about 200 mg/day of the drug conjugate, about 50 mg/day to about 300 mg/day of the drug conjugate, about 50 mg/day to about 500 mg/day of the drug conjugate, about 50 mg/day to about 1,000 mg/day of the drug conjugate, about 50 mg/day to about 2,000 mg/day of the drug conjugate, about 50 mg/day to about 5,000 mg/day of the drug conjugate, about 50 mg/day to about 10,000 mg/day of the drug conjugate, about 50 mg/day to about 20,000 mg/day of the drug conjugate, about 100 mg/day to about 200 mg/day of the drug conjugate, about 100 mg/day to about 300 mg/day of the drug conjugate, about 100 mg/day to about 500 mg/day of the drug conjugate, about 100 mg/day to about 1,000 mg/day of the drug conjugate, about 100 mg/day to about 2,000 mg/day of the drug conjugate, about 100 mg/day to about 5,000 mg/day of the drug conjugate, about 100 mg/day to about 10,000 mg/day of the drug conjugate, about 100 mg/day to about 20,000 mg/day of the drug conjugate, about 200 mg/day to about 300 mg/day of the drug conjugate, about 200 mg/day to about 500 mg/day of the drug conjugate, about 200 mg/day to about 1,000 mg/day of the drug conjugate, about 200 mg/day to about 2,000 mg/day of the drug conjugate, about 200 mg/day to about 5,000 mg/day of the drug conjugate, about 200 mg/day to about 10,000 mg/day of the drug conjugate, about 200 mg/day to about 20,000 mg/day of the drug conjugate, about 300 mg/day to about 500 mg/day of the drug conjugate, about 300 mg/day to about 1,000 mg/day of the drug conjugate, about 300 mg/day to about 2,000 mg/day of the drug conjugate, about 300 mg/day to about 5,000 mg/day of the drug conjugate, about 300 mg/day to about 10,000 mg/day of the drug conjugate, about 300 mg/day to about 20,000 mg/day of the drug conjugate, about 500 mg/day to about 1,000 mg/day of the drug conjugate, about 500 mg/day to about 2,000 mg/day of the drug conjugate, about 500 mg/day to about 5,000 mg/day of the drug conjugate, about 500 mg/day to about 10,000 mg/day of the drug conjugate, about 500 mg/day to about 20,000 mg/day of the drug conjugate, about 1,000 mg/day to about 2,000 mg/day of the drug conjugate, about 1,000 mg/day to about 5,000 mg/day of the drug conjugate, about 1,000 mg/day to about 10,000 mg/day of the drug conjugate, about 1,000 mg/day to about 20,000 mg/day of the drug conjugate, about 2,000 mg/day to about 5,000 mg/day of the drug conjugate, about 2,000 mg/day to about 10,000 mg/day of the drug conjugate, about 2,000 mg/day to about 20,000 mg/day of the drug conjugate, about 5,000 mg/day to about 10,000 mg/day of the drug conjugate, about 5,000 mg/day to about 20,000 mg/day of the drug conjugate, or about 10,000 mg/day to about 20,000 mg/day of the drug conjugate.

In some embodiments, the dose of the crystalline forms of Formula I and/or pharmaceutical compositions thereof may be adjusted to provide about 0.5 mg/day of the drug conjugate, about 5 mg/day of the drug conjugate, about 50 mg/day of the drug conjugate, about 100 mg/day of the drug conjugate, about 200 mg/day of the drug conjugate, about 300 mg/day of the drug conjugate, about 500 mg/day of the drug conjugate, about 1,000 mg/day of the drug conjugate, about 2,000 mg/day of the drug conjugate, about 5,000 mg/day of the drug conjugate, about 10,000 mg/day of the drug conjugate, or about 20,000 mg/day of the drug conjugate.

In some embodiments, the dose of the crystalline forms of the compound of Formula I and/or pharmaceutical compositions thereof may be adjusted to provide between about 50 mg/day and about 2000 mg/day of drug conjugate (equivalent to about 25 mg/day naproxen, 17 mg/day pregabalin and about 1000 mg/day of naproxen, 690 mg/day of pregabalin).

Dosage ranges may be readily determined by methods known to the skilled artisan.

The crystalline forms of the compound of Formula I and/or pharmaceutical compositions thereof may be assayed *in vitro* and *in vivo*, for the desired therapeutic or prophylactic activity, prior to use in humans. In some embodiments, a therapeutically effective dose of the crystalline forms of the compound of Formula I and/or pharmaceutical compositions thereof described herein will provide therapeutic benefit without causing substantial toxicity. Toxicity of crystalline forms of the compound of Formula I and/or pharmaceutical compositions thereof may be determined using standard pharmaceutical procedures. The dose ratio between toxic and therapeutic effect is the therapeutic index. The dosage of crystalline forms of Formula I and/or pharmaceutical compositions thereof described herein may be within a range of circulating concentrations that include an effective dose with little or no toxicity.

### Combination Therapy

In certain embodiments, crystalline forms of the compound of Formula I and/or pharmaceutical compositions thereof can be used in combination therapy with at least one other therapeutic agent. The crystalline forms of the compound of Formula I and/or pharmaceutical compositions thereof and the other therapeutic agent can act additively or synergistically. In some embodiments, the crystalline forms of the compound of Formula I and the other therapeutic agent act additively. In some embodiments, the crystalline forms of Formula I and the other therapeutic agent act synergistically.

In some embodiments, a pharmaceutical composition comprising the crystalline forms of Formula I is administered concurrently with the administration of another therapeutic agent, which can be part of the same pharmaceutical composition as the crystalline forms of Formula I or a different pharmaceutical composition. In other embodiments, a pharmaceutical composition comprising the crystalline forms of Formula I is administered prior or subsequent to administration of another therapeutic agent.

In some examples, a crystalline form of the compound of Formula I is administered in combination with an amorphous form of the compound of Formula I, another crystalline forms of Formula I, naproxen and/or pregabalin.

### Embodiments

Embodiment 1: A composition comprising a crystalline form of a compound of Formula I:

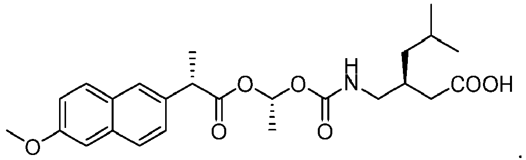

Embodiment 2: The composition of embodiment 1, wherein the crystalline form is characterized by having X-ray powder diffraction (XRPD) peaks at about 8.6, 15.5, 16.4, 17.9, and 20.6 degrees 2Θ.

Embodiment 3: The composition of embodiment 2, wherein the crystalline form is characterized by having at least one additional XRPD peak at about 6.4, 17.2, 17.5, 20.4, 21.4, 22.4, 23.7, 23.9, or 27.6 degrees 20

Embodiment 4: The composition of embodiment 1, wherein the crystalline form is characterized by having XRPD peaks at about 6.4, 8.6, 15.5, 16.4, 17.2, 17.5, 17.9, 20.4, 20.6, 21.4, 22.4, 23.7, 23.9, and 27.6 degrees 2Θ.

Embodiment 5: The composition of any one of embodiments 2 to 4, wherein the crystalline form is characterized by having at least one additional XRPD peak at about 9.3, 9.8, 19.9, 21.7, 22.9, 24.3, 25.0, 25.6, 26.4, 26.9, 29.7 or 31.3 degrees 2Θ.

Embodiment 6: The composition of any one of the preceding embodiments, wherein the crystalline form is characterized by having XRPD peaks at about 6.4, 8.6, 9.3, 9.8, 15.5, 16.4, 17.2, 17.5, 17.9, 19.9, 20.4, 20.6, 21.4, 21.7, 22.4, 22.9, 23.7, 23.9, 24.3, 25.0, 25.6, 26.4, 26.9, 27.6, 29.7 and 31.3 degrees 2Θ.

Embodiment 7: The composition of any one of the preceding embodiments, wherein the crystalline form has a chemical purity of greater than about 90%.

Embodiment 8: The composition of embodiment 7, wherein the chemical purity of the crystalline from is determined by HPLC analysis.

Embodiment 9: The composition of any one of the preceding embodiments, wherein the crystalline from has an enantiomeric purity of greater than about 90%.

Embodiment 10: The composition of any one of the preceding embodiments, wherein the crystalline from has a diastereomeric purity of greater than about 90%.

Embodiment 11: The composition of any one of the preceding embodiments, wherein the crystalline form has a melting point in the range of from about 100° C to about 140° C.

Embodiment 12: The composition of any one of the preceding embodiments, wherein the crystalline form has a melting point in the range of from about 110 °C to about 130 °C.

Embodiment 13: The composition of any one of the preceding embodiments, wherein the crystalline form has a melting point in the range of from about from about 118 °C to about 121 °C.

Embodiment 14: The composition of any one of the preceding embodiments, wherein the crystalline form has a melting point in the range of from about from about 119 °C to about 121 °C.

Embodiment 15: The composition of any one of the preceding embodiments, wherein the composition comprises at least two crystalline forms of the compound of Formula I.

Embodiment 16: A pharmaceutical composition comprising the composition of any one of the preceding embodiments.

Embodiment 17: The pharmaceutical composition of embodiment 16, wherein the pharmaceutical composition is in a solid dosage form.

Embodiment 18: The pharmaceutical composition of embodiment 16 or 17, wherein the pharmaceutical composition is a capsule.

Embodiment 19: The pharmaceutical composition of embodiment 16, wherein the pharmaceutical composition is a suspension.

Embodiment 20: The pharmaceutical composition of embodiment 19, wherein the pharmaceutical composition is an aqueous suspension.

Embodiment 21: The pharmaceutical composition of embodiment 16, wherein the pharmaceutical composition is a liquid.

Embodiment 22: The pharmaceutical composition of embodiment 21, wherein the pharmaceutical composition comprises a solution of the crystalline form in water.

Embodiment 23: The pharmaceutical composition of embodiment 21, wherein the pharmaceutical composition comprises a solution of the crystalline form in a saline solution, an aqueous dextrose solution, a glycerol solution, or a combination thereof.

Embodiment 24: The pharmaceutical composition of embodiment 22 or 23, wherein the pharmaceutical composition is for intravenous administration.

Embodiment 25: The pharmaceutical composition of any one of embodiments 16 to 23, wherein the pharmaceutical composition is for oral administration.

Embodiment 26: The pharmaceutical composition of any one of embodiments 16 to 25, further comprising one or more excipients selected from the group consisting of wetting agents, emulsifying agents, buffering agents, stabilizing agents, thickening agents, lubricating agents, and coloring agents.

Embodiment 27: The pharmaceutical composition of any one of embodiments 16 to 26, wherein the pharmaceutical composition comprises at least two crystalline forms of the compound of Formula I.

Embodiment 28: A kit for preventing or treating a disease in a subject, the kit comprising the composition of any one of embodiments 1 to 15, or the pharmaceutical composition of any one of embodiments 16 to 27, and instructions for using the kit.

Embodiment 29: The kit of embodiment 28, wherein the subject is an animal.

Embodiment 30: The kit of embodiment 28 or 29, wherein the subject is a human.

Embodiment 31: The kit of any one of embodiments 28 to 30, wherein the disease is a pain or an inflammatory disease.

Embodiment 32: The kit of embodiment 31, wherein the pain is a neuropathic pain or a musculoskeletal pain.

Embodiment 33: The kit of embodiment 31, wherein the inflammatory disease is arthritis.

Embodiment 34: The kit of any one of embodiments 28 to 33, wherein the kit further comprises at least one additional therapeutic agent.

Embodiment 35: The kit of embodiment 34, wherein the at least one additional therapeutic agent is an agent for treatment of a pain or a neurological disease.

Embodiment 36: The kit of embodiment 34 or 35, wherein the composition or the pharmaceutical composition and the at least one additional therapeutic agent act additively.

Embodiment 37: The kit of embodiment 34 or 35, wherein the composition or the pharmaceutical composition and the at least one additional therapeutic agent act synergistically.

Embodiment 38: A method of treating or preventing a disease in a subject in need thereof, wherein the method comprises administering to the subject an effective amount of the composition of any one of embodiments 1 to 15 or the pharmaceutical composition of any one of embodiments 16 to 27.

Embodiment 39: The method of embodiment 38, wherein the subject is a human.

Embodiment 40: The method of embodiment 38 or 39, wherein the disease is a pain or an inflammatory disease.

Embodiment 41: The method of embodiment 40, wherein the pain is a neuropathic pain or a musculoskeletal pain.

Embodiment 42: The method of embodiment 40, wherein the inflammatory disease is arthritis.

Embodiment 43: The method of any one of embodiments 38 to 42, wherein the method further comprises administering at least one additional therapeutic agent to the subject.

Embodiment 44: The method of embodiment 43, wherein the at least one additional therapeutic agent is an agent for treatment of a pain or a neurological disease.

Embodiment 45: The method of embodiment 43 or 44, wherein the composition or the pharmaceutical composition and the at least one additional therapeutic agent act additively.

Embodiment 46: The method of embodiment 43 or 44, wherein the composition or the pharmaceutical composition and the at least one additional therapeutic agent act synergistically.

Embodiment 47: The method of any one of embodiment 43 to 46, wherein the composition or the pharmaceutical composition and the at least one additional therapeutic agent are administered concurrently.

Embodiment 48: A method of making a crystalline form of a compound of Formula I: 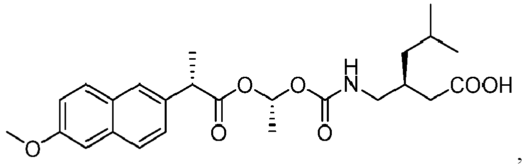 wherein the method comprises:
dissolving a composition comprising the compound of Formula I in a solvent to obtain a solution of the compound of Formula I; and
isolating the crystalline form from the solution of the compound of Formula I.

Embodiment 49: The method of embodiment 48, wherein the crystalline form is characterized by having XRPD peaks at about 8.6, 15.5, 16.4, 17.9, and 20.6 degrees 2Θ.

Embodiment 50: The method of embodiment 49, wherein the crystalline form is characterized by having at least one additional XRPD peak at about 6.4, 17.2, 17.5, 20.4, 21.4, 22.4, 23.7, 23.9, or 27.6 degrees 2Θ.

Embodiment 51: The method of embodiment 48, wherein the crystalline form is characterized by having XRPD peaks at about 6.4, 8.6, 15.5, 16.4, 17.2, 17.5, 17.9, 20.4, 20.6, 21.4, 22.4, 23.7, 23.9, and 27.6 degrees 2Θ.

Embodiment 52: The method of any one of embodiments 49 to 51, wherein the crystalline form is characterized by having at least one additional XRPD peak at about 9.3, 9.8, 19.9, 21.7, 22.9, 24.3, 25.0, 25.6, 26.4, 26.9, 29.7 or 31.3 degrees 2Θ.

Embodiment 53: The method of any one of embodiments 48 to 52, wherein the step of dissolving comprises heating a mixture of the composition comprising the compound of Formula I and the solvent to a temperature above an ambient temperature.

Embodiment 54: The method of any one of embodiments 48 to 53, wherein the step of isolating the crystalline form from the solution of the compound of Formula I comprises cooling the solution of the compound of Formula I to a temperature below an ambient temperature.

Embodiment 55: The method of any one of embodiments 48 to 54, wherein the step of isolating the crystalline form from the solution of the compound of Formula I comprises cooling the solution of the compound of Formula I to a temperature of between about 0 °C and 25 °C.

Embodiment 56: The method of any one of embodiments 48 to 55, wherein the method further comprises adding an additional solvent to the solution of the compound of Formula I.

Embodiment 57: The method of any one of embodiment 48 to 56, wherein the solvent comprises a polar protic solvent.

Embodiment 58: The method of any one of embodiment 48 to 57, wherein the solvent comprises isopropanol.

Embodiment 59: The method of any one of embodiments 48 to 58, wherein the step of dissolving comprises heating a mixture of the composition comprising the compound of Formula I and the solvent to a temperature of about 40 °C to about reflux temperature.

Embodiment 60: The method of any one of embodiments 57 to 59, wherein the additional solvent comprises an alkane.

Embodiment 61: The method of any one of embodiments 57 to 60, wherein the additional solvent comprises heptane.

Embodiment 62: The method of any one of the embodiments 56 to 61, wherein the solvent and the additional solvent are used in a volume ratio of about 1:2 to about 1:3 (v/v).

Embodiment 63: The method of any one of embodiment 48 to 56, wherein the solvent comprises heptane, ethyl acetate, or a mixture thereof.

Embodiment 64: The method of embodiment 63, wherein the solvent comprises heptane and ethyl acetate in ratio of about 10:1 by volume.

Embodiment 65: The method of embodiment 63 or 64, wherein the step of dissolving comprises heating a mixture of the composition comprising the compound of Formula I and the solvent to a temperature of about 50 °C to about reflux temperature.

Embodiment 66: The method of any one of embodiments 63 to 65, wherein the step of dissolving comprises heating a mixture of the composition comprising the compound of Formula I and the solvent to a temperature of about 70 °C.

Embodiment 67: The method of any one of embodiment 48 to 56, wherein the solvent comprises methylcyclohexane, methyl *t*-butyl ether, or a mixture thereof.

Embodiment 68: The method of embodiment 67, wherein the solvent comprises methylcyclohexane and methyl *t*-butyl ether in ratio of about 10:1 by volume.

Embodiment 69: The method of embodiment 67 or 68, wherein the step of dissolving comprises heating a mixture of the composition comprising the compound of Formula I and the solvent to a temperature of about 20 °C to about 40 °C.

Embodiment 70: The method of any one of embodiments 48 to 69, further comprising introducing a seed crystalline form into the solution of the compound of Formula I.

While preferred embodiments of the present invention have been shown and described herein, it will be apparent to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### EXAMPLES

The following examples describe in detail the preparation of the compound of Formula I and the crystalline forms thereof. It will be apparent to those skilled in the art that many modifications, both to materials and methods, may be practiced without departing from the scope of the invention.

### Example 1: Exemplary synthesis of the compound of Formula I and crystallization thereof

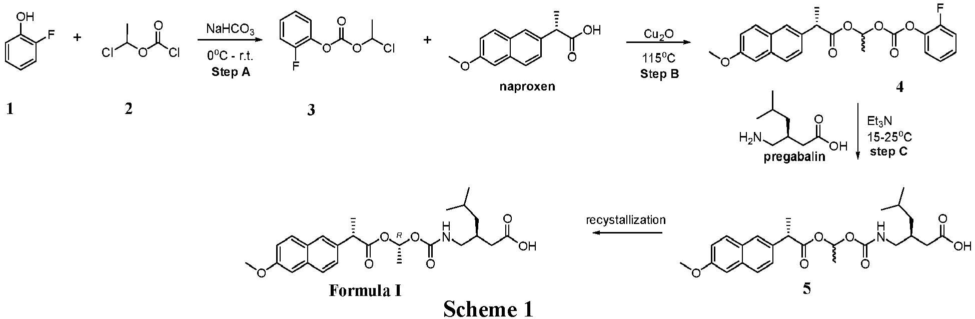

### Step A: Synthesis of 1-chloroethyl 2-fluorophenyl carbonate (3)

A suitable reaction vessel was charged with water and sodium bicarbonate followed by the starting material 2-fluoro-phenol (1). The mixture was cooled to a temperature of about 0~5°C and 1-chloroethyl chloroformate (2) was added slowly while maintaining the temperature at 0~5°C. The temperature was raised to about 15 ± 5°C. When the reaction was judged complete by the disappearance of 2-fluoro-phenol (criteria: ≤ 2.0%, by HPLC) the reaction was worked up. *n*-Heptane was added and the organic phase was separated, washed with water and brine. The solution was concentrated, then toluene was added and the solution was concentrated again. The toluene addition and the concentration cycle were repeated once more.

### Step B: Synthesis of (S)-((R,S)-1-((2-fluorophenoxy)carbonyloxy)ethyl 2-(6-methoxynaphthalen-2-yl)propanoate (4)

To a solution of naproxen in toluene in a suitable reaction vessel, 1-chloroethyl 2-fluorophenyl carbonate (3) and cuprous oxide was added. The temperature of the mixture was raised to about 115 ± 5°C. When the reaction was judged complete by the disappearance of 1-chloroethyl 2-fluorophenyl carbonate (criteria: ≤ 2.5%, by HPLC) the reaction was worked up. Methyl *tert*-butyl ether was added at about 50 ± 5°C. The resulting mixture was filtered and the filtrate was collected at about 25 ± 5°C. Purified water was added into the filtrate and then the mixture was cooled to about 0 ± 5°C. The mixture was alkalified with ammonium hydroxide to a pH of about 9~11 and the organic phase was separated and washed with ammonium hydroxide and brine. The solution was concentrated, then acetonitrile was added and the solution was concentrated again. The acetonitrile addition and the concentration cycle were repeated some more times until the residual toluene was not more than 10% (by GC method).

### Step C: Synthesis of (S)-3-((((R)-1-((S)-2-(6-methoxynaphthalen-2-yl)propanoyloxy)ethoxy) carbonylamino)methyl)-5-methylhexanoic acid (the compound of Formula I)

To a solution of the mixture of (*S*)-((*R,S*)-1-((2-fluorophenoxy)carbonyloxy)ethyl 2-(6-methoxynaphthalen-2-yl)propanoate (**4**) in acetonitrile and methyl *tert*-butyl ether in a suitable reaction vessel, purified water and pregabalin was charged. Triethylamine was added slowly while maintaining the temperature at about 15 ± 5°C. The temperature was raised to about 25 ± 3°C for reaction. When the reaction was judged complete by the disappearance of (*S*)-((*R,S*)-1-((2-fluorophenoxy)carbonyloxy)ethyl 2-(6-methoxynaphthalen-2-yl)propanoate (criteria: ≤ 0.5%, by HPLC) the reaction was worked up. The resulting mixture was acidified with KHSO₄ to pH 3-5 and extracted with methyl *tert*-butyl ether. The combined organic layers were washed with purified water and brine. The organic phase was then concentrated. Isopropanol was added and the solution was concentrated again. The isopropanol addition and the concentration cycle was repeated once or more times until the total residual acetonitrile and methyl *tert*-butyl ether was not more than 5% (by GC method). n-Heptane was added into the mixture at about 40-45 °C and stirred and then the temperature was gradually lowered at set appropriate intervals to crystallize (*S*)-3-((((*R*)-1-((*S*)-2-(6-methoxynaphthalen-2-yl)propanoyloxy)ethoxy) carbonylamino)methyl)-5-methylhexanoic acid (**5**) from the system. When the precipitation was complete, the heterogeneous mixture was centrifuged and the solid was collected.

The crude product of (*S*)-3-((((*R*)-1-((*S*)-2-(6-methoxynaphthalen-2-yl)propanoyloxy)ethoxy) carbonyl-amino)methyl)-5-methylhexanoic acid (**5**) was added to a solution of isopropanol and water in a suitable vessel. The mixture was stirred while raising the temperature to 45 ± 3 °C until all the solid was dissolved, then the temperature was lowered gradually at set appropriate intervals to recrystallize (*S*)-3-((((*R*)-1-((*S*)-2-(6-methoxynaphthalen-2-yl)propanoyloxy)ethoxy) carbonyl-amino)methyl)-5-methylhexanoic acid from the system. When the precipitation had stopped, the heterogeneous mixture was centrifuged and the expected pure (*S*)-3-((((*R*)-1-((*S*)-2-(6-methoxynaphthalen-2-yl)propanoyloxy)ethoxy) carbonyl-amino)methyl)-5-methylhexanoic acid (**Formula I**) was collected.

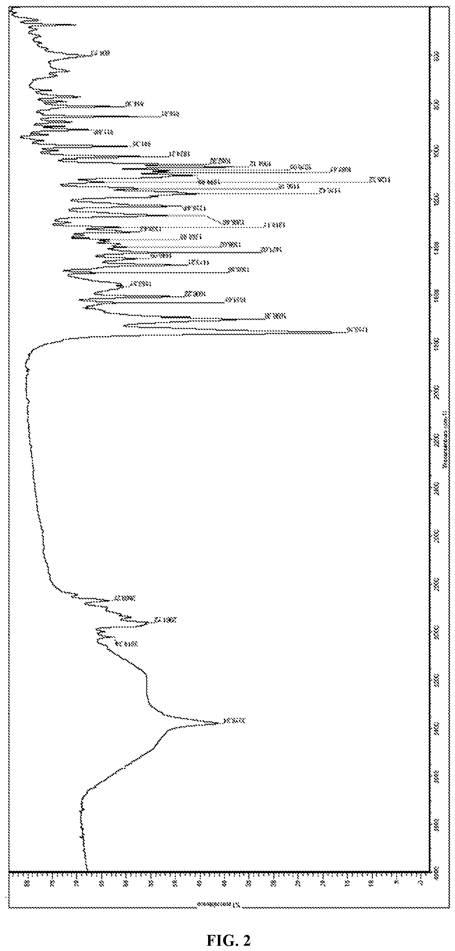

The FT-IR (Fourier Transform Infrared Spectroscopy) of (*S*)-3-((((*R*)-1-((*S*)-2-(6-methoxynaphthalen-2-yl)propanoyloxy)ethoxy) carbonyl-amino)methyl)-5-methylhexanoic acid was carried out using IS10 Fourier Transform Infrared Spectrometer (Thermo). The samples were mixed with dry, powdered potassium bromide (KBr) to create a mixture containing approximately 1% sample by weight. The mixture was compacted into a disk in vacuum under high pressure. Assignments for the major absorbance frequencies are listed in Table 1 and the FT-IR Spectrum is shown in FIG. 2.

**Table 1. Assignments for the major absorbance frequencies in the FT-IR Spectrum of (S)-3-((((R)-1-((S)-2-(6-methoxynaphthalen-2-yl)propanoyloxy)ethoxy) carbonyl-amino)methyl)-5-methylhexanoic acid c**

| **Frequency (cm⁻¹)** | **Vibrational Mode** | **Assignment** |
|---|---|---|
| 3500-3100 | v_{O-H} | -OH |
| 3378 | v_{N-H} | -NH |
| 3019 | v_{=C-H} | =C-H |
| 2962, 2869 | v_{C-H} | -C-H |
| 1754, 1698, 1632 | v_{C=O} | C=O |
| 1606, 1506, 1446 | v_{C=C} | Aromatic ring |
| 1268, 1228 | v_{C-O-C} | -C(=O)-O-C |

The NMR spectrum (¹H-NMR, ¹³C-NMR) of (*S*)-3-((((*R*)-1-((*S*)-2-(6-methoxynaphthalen-2-yl)propanoyloxy)ethoxy) carbonyl-amino)methyl)-5-methylhexanoic acid was obtained as a solution in DMSO-*d6* using a Bruker Altra Shield TM 400 NMR. ¹H-NMR (DMSO-*d6*, 400MHz): δ 7.78 (d, J = 8.8 Hz, 1H), 7.76 (d, J = 7.2 Hz, 1H), 7.69 (brs, 1H), 7.35 (overlap, 1H), 7.28 (d, J = 2.4 Hz, 1H), 7.15 (d, J = 8.8, 2.4 Hz, 1H), 6.73 (q, J = 5.2 Hz, 1H), 3.90 (q, J = 7.2 Hz, 1H), 3.87 (s, 3H), 2.89 (m, 1H), 2.82 (m, 1H), 2.17 (dd, J = 15.6, 5.6 Hz, 1H), 1.99 (dd, J = 15.6, 7.2 Hz, 1H), 1.89 (m, 1H), 1.57 (hept, J = 6.8 Hz, 1H), 1.45 (d, J = 7.2 Hz, 3H), 1.38 (d, J = 5.6 Hz, 3H), 1.05 (m, 1H), 0.98 (m, 1H), 0.80 (d, J = 6.8 Hz, 3H), 0.80 (d, J = 6.8 Hz, 3H). ¹³C NMR (DMSO-d6, 101 MHz) δ 174.35, 172.60, 157.67, 154.49, 135.70, 133.75,129.64, 128.83, 127.36, 126.72, 126.18, 119.14, 106.12, 89.34, 55.60, 44.88, 44.02, 41.17, 37.43, 33.40, 25,08, 23.22, 22.90, 20.07, 18.92.

MS (Mass Spectroscopy) was carried out using Waters Acquity I Class UPLC with Xevo G2-XS QTof HRMS System with positive mode electrospray ionization. The sample was dissolved in acetonitrile with concentration of 0.1 mg/mL. The MS spectrum showed an ion peak of [M+Na]⁺ at m/z 482.2154 (calculated value:482.2155, C₂₅H₃₃NO₇.Na).

### Example 2: Alternative synthetic route of (S)-3-((((R)-1-((S)-2-(6-methoxynaphthalen-2-yl)propanoyloxy)ethoxy) carbonyl-amino)methyl)-5-methylhexanoic acid

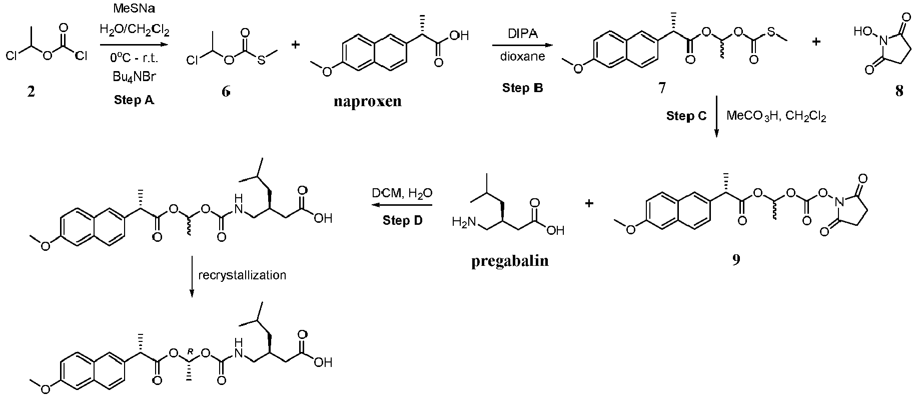

### Step A : Synthesis of O-1-Chloroethyl S Methyl Carbonothioate (6)

Under N₂ atmosphere pressure, DCM (2.0 vol.), 1-chloroethyl chloroformate (1.0 eq.) (**2**) and TBAB (0.01 eq.) were added to a reactor and the temperature was cooled to about 0 ± 5°C. NaSMe (aq., 20%, w/w) (1.15 eq.) was added dropwise to the reactor at about 0 ± 5°C. The reaction mixture was sampled for GC analysis after stirring for 4 hours at about 0 ± 5°C until 1-chloroethyl chloroformate is less than 1.5% (peak area) in the chromatogram. The deionized water (2.0 vol.) was added into the system to wash the mixture for three times, and the organic phases were separated and collected. The organic phase was further washed with 5% aq. NaCl solution (3.0 vol.), then separated and collected. The organic phase was concentrated under reduced pressure below 20 °C until the residual volume was 1.0 ± 0.2 vol. The reaction product *O*-1-chloroethyl *S*-methyl carbonothioate (**6**) was sampled for the GC analysis. The GC purity of *O*-1-chloroethyl *S*-methyl carbonothioate is ≥ 95%.

### Step B : Synthesis of (S)-((R,S)-1-(methylthiocarbonyloxy)ethyl 2-(6-methoxynaphthalen-2-yl)propanoate (7)

Under a nitrogen atmosphere, O-1-chloroethyl *S*-methyl carbonothioate (1.0 eq.), naproxen (1.1 eq.) and KI (0.1 eq.) were dissolved in 1,4-dioxane (2.0 vol.) in a reactor. The mixture was heated to about 50 ± 5°C. DIPA (1.0 eq.) was added dropwise to the reactor and the temperature was kept below about 60°C, followed by stirring of the mixture for 20-30 mins. Then the reaction temperature was brought to about 75 ± 5°C. A sample of the reaction mixture was taken for HPLC analysis every 2.0 ± 0.2 hour for 20 hours at about 75 ± 5°C. The reaction was cooled to about 25 ± 5°C after naproxen is ≤ 30% (peak area) measured by HPLC. Then deionized water (8.0 vol.) and MTBE (8.0 vol.) was added into the reaction mixture and the mixture stirred. Stirring was stopped, then the layers were separated and the organic phase was collected. The organic phase was washed with the deionized water (8.0 vol.), separated and collected. The organic phase was then washed with 18% K₂CO₃ aq. solution (wt./wt., 8.0 vol.) for two times. Separate and collect the organic phase. The mixture was analyzed by HPLC until naproxen is < 1.0% (peak area) based on the HPLC.

Wash the organic phase with 15% NaCl aq. solution (5.0 vol.), separate and collect the organic phase. Concentrate the organic phase under reduced pressure below 40°C. Charged the reactor with heptane (5.0 vol.) and concentrate the mixture again under reduced pressure below 40°C until the residual volume is 2-3 vol. This process was repeated once again. And then, a portion of heptane (5 vol.) was added into the reactor. The solution turned into a slurry and was stirred for 16 h at 20-25°C. The mixture was then filtered, and the solid was washed with heptane (0.5 vol.) twice. The solid sample was collected for HPLC analysis. The procedure is repeated till the purity of (*S*)-((*R,S*)-1-(methylthiocarbonyloxy)ethyl 2-(6-methoxynaphthalen-2-yl)propanoate (**7**) was ≥ 95%. The solid product was dried under vacuum at 30 ± 5°C. The isolated product was the desired diastereomer and had the following characteristics: mp: 65-66 °C. IR (KBr) *v*ₘₐₓ: 3055, 2986, 2937, 2916, 2848, 1750, 1719, 1606, 1451, 1392, 1264, 1177, 1134, 1050, 910, 854, 747 cm⁻¹. ¹H NMR (400 MHz, CDCl₃) δ 7.56 (d, *J* = 2.8 Hz, 1H), 7.54 (t, *J* = 2.1 Hz, 1H), 7.51 (s, 0.5H), 7.50 (s, 0.5H),7.26 - 7.24 (m, 0.5H), 7.24 - 7.21 (m, 0.5H), 7.01 (t, *J* = 2.2 Hz, 0.5H), 6.99 (t, *J* = 2.2 Hz, 0.5H), 6.96 (s, 1H), 6.85 (dq, *J* = 10.9, 5.4 Hz, 1H), 3.75 - 3.67 (m, 4H), 2.14 (s, 1.5H), 1.98 (s, 1.5H), 1.44 (d, *J* = 1.6 Hz, 1.5H), 1.43 (d, *J* = 1.6 Hz, 1.5H), 1.33 (d, *J* = 5.5 Hz, 1.5H), 1.24 (d, *J* = 5.5 Hz, 1.5H). ¹³C NMR (101 MHz, CDCl₃) δ 171.43, 171.32, 169.00, 168.80, 156.64, 156.60, 133.92, 133.79, 132.70, 132.66, 128.26, 128.21, 127.85 (2), 126.16, 126.08, 125.14, 125.02, 124.97 (2), 117.97, 117.85, 104.49, 104.46, 89.31, 89.05, 54.12 (2), 44.15, 44.11, 18.34, 18.24, 17.38, 17.18, 12.15, 11.97.

### Step C : Synthesis of (S)-((R,S)-1-((2,5-dioxopyrrolidin-1-yloxy)carbonyloxy)ethyl) 2-(6-methoxynaphthalen-2-yl)propanoate (9)

Under N₂ atmosphere, (*S*)-((*R,S*)-1-(methylthiocarbonyloxy)ethyl 2-(6-methoxynaphthalen-2-yl)propanoate (1.0 eq.) (**7**) and NHS (20.0 eq.) (**8**) were dissolved in DCM (10.0 vol.) in a reactor. Peracetic acid solution that was prepared 48 hours earlier (15%wt) (2.5 eq.) was added dropwise to the reactor within 2-3 hours below 25°C. The mixture was continued to stir for additional 1-2 hours at 15 ± 5°C, then the reaction mixture was allowed to warm up to 20~25°C. The reaction mixture was sampled for HPLC analysis after stirring for 6 hours at 20-25°C. Stirring continued until the (*S*)-((*R,S*)-1-(methylthiocarbonyloxy)ethyl 2-(6-methoxynaphthalen-2-yl)propanoate was ≤ 5.0% based on the peak area.

A portion of 5% NaCl (5.0 vol.) aqueous solution was added to the reactor. The layers were separated, and the organic phase was collected. The organic phase was washed with 5% NaHCO₃ (5.0 vol.) for several times until naproxen is ≤ 1.5% (peak area) in the HPLC chromatogram.

The organic phase was washed with 10% Na₂S₂O₃ (5.0 vol.) aq. solution. The phases were separated, and the organic phase was collected. The organic phase was filtered through celatom. The filter cake was washed with DCM (1.0 vol.) twice, and the filtrate was combined and collected. The filtrate was further washed with 15% NaCl (5.0 vol.), then the organic phase was separated and concentrated. The crude (*S*)-((*R,S*)-1-((2,5-dioxopyrrolidin-1-yloxy)carbonyloxy)ethyl) 2-(6-methoxynaphthalen-2-yl)propanoate) (**9**) was carried to the next step directly. The desired diastereomer resulted and had the following characteristics: white crystal, mp: 145-146 °C. IR (KBr) *v*ₘₐₓ: 2992, 2942, 1819, 1792, 1744, 1632, 1606, 1506, 1486, 1454, 1393, 1373, 1260, 1234, 1202, 1048, 910, 879, 812, 735, 644 cm⁻¹. ¹H NMR (400 MHz, CDCl₃) δ 7.71 (dd, *J* = 8.5, 4.5 Hz, 2H), 7.65 (s, 1H), 7.38 (s, 0.5H), 7.36 (s, 0.5H), 7.17 - 7.08 (m, 2H), 6.88 - 6.79 (m, 1H), 3.98 - 3.83 (m, 4H), 2.82 (s, 2H), 2.73 (s, 2H), 1.58 (m, 4.5H), 1.48 (d, *J* = 5.4 Hz, 1.5H). ¹³C NMR (101 MHz, CDCl₃) δ 172.31, 172.18, 168.39 (2), 168.24 (2), 157.75, 157.68, 149.87, 149.73, 134.66, 134.40, 133.80, 133.74, 129.45, 129.33, 128.93, 128.90, 127.36, 127.32, 126.18, 126.15 (2), 126.09, 119.09, 118.85, 105.58 (2), 93.89, 93.79, 55.33 (2), 45.27, 45.04, 25.44 (2), 25.29 (2), 19.27, 19.19, 18.41, 18.34. HRMS (ESI) found 438.1157, ([M + Na]⁺, calcd for C₂₁H₂₁NO₈ 438.1159).

### Step D : Synthesis of (S)-3-((((R)-1-((S)-2-(6-methoxynaphthalen-2-yl)propanoyloxy)ethoxy) carbonylamino)methyl)-5-methylhexanoic acid(Formula I)

Under N₂, the crude (*S*)-((*R,S*)-1-((2,5-dioxopyrrolidin-1-yloxy)carbonyloxy)ethyl) 2-(6-methoxynaphthalen-2-yl)propanoate (**9**) (theoretical yield × Purity by HPLC, 1.0 eq.) was dissolved in DCM (10.0 vol.) in a reactor. Pregabalin (1.0 eq.) and deionized water (1.5 vol.) were added to the reactor. The reaction mixture was stirred at 25 ± 3°C before it was sampled for HPLC analysis. A portion of deionized H₂O (5.0 vol.) was then added to the reactor when the peak area of (*S*)-((*R,S*)-1-((2,5-dioxopyrrolidin-1-yloxy)carbonyloxy)ethyl) 2-(6-methoxynaphthalen-2-yl)propanoate was ≤ 1.0% in the HPLC analysis. The layers were separated, and the organic phase was collected. The organic phase was further washed with deionized H₂O (5.0 vol.), followed by 15% NaCl (5.0 vol.) aq. solution. The layers were separated, and the organic phase was collected and concentrated.

The crude (*S*)-3-((((*R*,*S*)-1-((*S*)-2-(6-methoxynaphthalen-2-yl)propanoyloxy)ethoxy) carbonylamino)methyl)-5-methylhexanoic acid (theoretical production × purity, 1.0 eq.) was mixed with silica gel (100-200 mesh) (2.0 wt.%). This mixture was then loaded onto the silica gel (100-200 mesh, 5.5 wt.%) packing column with a mixture of heptane (165 vol.) and EtOAc (50 vol.) as mobile phase. The fractions of (*S*)-3-((((*R*,*S*)-1-((*S*)-2-(6-methoxynaphthalen-2-yl)propanoyloxy)ethoxy) carbonyl-amino)methyl)-5-methylhexanoic acid with higher than 90% HPLC purity were collected, and concentrated under reduced pressure below 45°C.

Under N₂ atmosphere, (*S*)-3-((((*R*,*S*)-1-((*S*)-2-(6-methoxynaphthalen-2-yl)propanoyloxy)ethoxy) carbonyl-amino)methyl)-5-methylhexanoic acid (1.0 eq.) was dissolved in IPA (2.0 vol.) in a reactor. The mixture was heated to about 40 ± 5°C and stirred to achieve a clear solution. Heptane (5.5 vol.) was added to the reactor dropwise and the mixture was stirred for 1.0 hour. The mixture was cooled to 20 ± 5°C and stirred for 1 hour. It was then cooled to about 5 ± 5°C and stirred for 15 hours. The heterogeneous mixture was filtered and the resulting solid was washed with heptane (0.5 vol.) twice. The solid (S)-3-((((R)-1-((S)-2-(6-methoxynaphthalen-2-yl)propanoyloxy)ethoxy) carbonyl-amino)methyl)-5-methylhexanoic acid (Formula I )was collected.

### Example 3: Differential Scanning Calorimetry analysis of the crystalline form of Formula I

The Differential Scanning Calorimetry curve of the crystalline form of Formula I was carried out using TA-Q200 Differential Scanning Calorimetric Analyzer. The Sample purge flow was at 50 mL/min and the heating rate was 10 °C/min. The sample cells upon equilibration temperature was 25 °C and the final temperature was set at 300 °C. The spectrum showed a sharp endothermic transition (melting point) at 119.90 °C with an onset temperature of 117.88 °C and a ΔH of 127.7 J/g. At the peak endotherm of 119-120 °C, the sample visibly melted. An overlap of the DSC and TGA curves is given in FIG. 3. Combined the results of DSC and TGA, confirmed the crystallinity of the crystalline form of Formula I.

### Example 4: Determination of the Stereochemistry

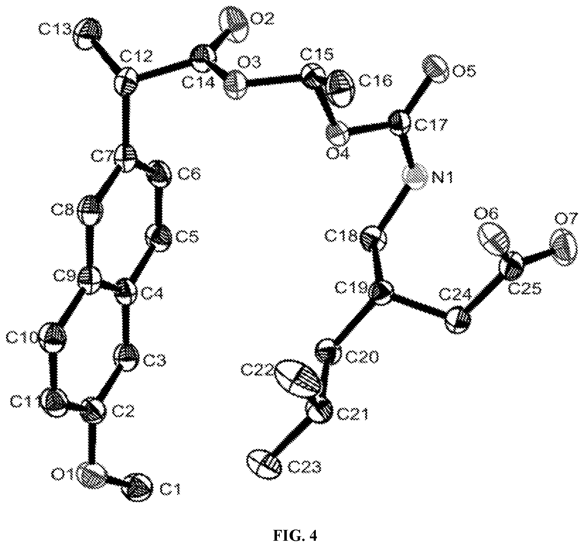

(*S*)-3-((((*R*)-1-((*S*)-2-(6-methoxynaphthalen-2-yl)propanoyloxy)ethoxy)carbonyl-amino)methyl)-5-methylhexanoic acid contains three chiral centers. The *S*-configuration of the naproxen moiety is controlled through the enantiomeric purity of starting material of *S*-naproxen, and the *S*-configuration of the pregabalin moiety is controlled through the enantiomeric purity of the starting material of *S-*Pregabalin during synthesis. The newly formed acyloxy carbon of the linker moiety has the *R-*stereochemical configuration in the major diastereomer that is isolated. An unambiguous assignment of acyloxy carbon configuration for the major diastereomer (crystallized) has been made *via* single crystal X-ray diffraction analysis. Data information of the single crystal X-ray diffraction and the confirmed stereo structure are given below and the X-ray structure of the compound of Formula I is shown in FIG. 4.

**Table 2: X-ray Crystallographic Data of (S)-3-((((R)-1-((S)-2-(6-methoxynaphthalen-2-yl)propanoyloxy) ethoxy) carbonyl-amino)methyl)-5-methylhexanoic acid**

| | |
|---|---|
| Empirical formula | C₂₅H₃₃NO₇ |
| Formula weight | 459.52 |
| Temperature | 293(2) |
| Wavelength | 0.71073 A |
| Crystal system, space group | Triclinic, P1 |
| Unit cell dimensions | a = 20.506(4) Å α= 90° |
| | b = 18.967(4) Å β= 90° |
| | c = 6.2866(13) Å γ= 90° |
| Volume | 2445.1(9) Å³ |
| Z, Calculated density | 4, 1.248 Mg/m' |
| Absorption coefficient | 0.091 mm⁻¹ |
| F(000) | 984 |
| Crystal size | 0.24*0.22*0.18 mm' |
| Theta range for data collection | 2.15 to 25.02°. |
| Limiting indice | -24<=h<=24, -22<=k<=20, -7<=1<=6 |
| Reflections collected / unique | 19364 / 4319 [R(int) = 0.0454] |
| Completeness to theta = 25.01 | 99.8 % |
| Absorption correction | Semi-empirical from equivalents |
| Max. and min. transmission | 0.9838 and 0.9785 |
| Refinement method | Full-matrix least-squares on F² |
| Data / restraints / parameters | 4319/ 1 / 309 |
| Goodness-of-fit on F² | 1.027 |
| Final R indices [I>2sigma(I)] | R1 = 0.0447, wR2 = 0.1027 |
| R indices (all data) | R1 = 0.0532, wR2 = 0.1087 |
| Absolute structure parameter | 0.4(10) |
| Largest diff. peak and hole | 0.196 and -0.160 e. Å⁻³ |

### Example 5. Optical Rotation of the compound of Formula I

The observed specific rotation of the compound of Formula I (5 mg/mL, dissolved in MeOH) is about +31° to +33.0°.

The following numbered paragraphs set out preferred features and preferred combinations of features of the present invention:
1. A crystalline form of a compound of Formula I: 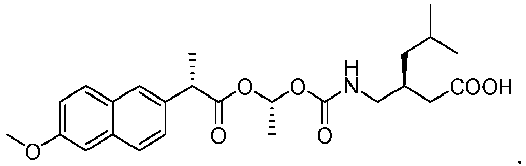
2. The crystalline form of paragraph 1, wherein the crystalline form is characterized by having X-ray powder diffraction (XRPD) peaks at about 8.6, 15.5, 16.4, 17.9, and 20.6 degrees 2θ.
3. The crystalline form of paragraph 2, wherein the crystalline form is characterized by having at least one additional XRPD peak at about 6.4, 17.2, 17.5, 20.4, 21.4, 22.4, 23.7, 23.9, or 27.6 degrees 2θ
4. The crystalline form of paragraph 1, wherein the crystalline form is characterized by having XRPD peaks at about 6.4, 8.6, 15.5, 16.4, 17.2, 17.5, 17.9, 20.4, 20.6, 21.4, 22.4, 23.7, 23.9, and 27.6 degrees 2θ.
5. The crystalline form of any one of paragraphs 2 to 4, wherein the crystalline form is characterized by having at least one additional XRPD peak at about 9.3, 9.8, 19.9, 21.7, 22.9, 24.3, 25.0, 25.6, 26.4, 26.9, 29.7 or 31.3 degrees 2θ.
6. The crystalline form of any one of the preceding paragraphs, wherein the crystalline form is characterized by having XRPD peaks at about 6.4, 8.6, 9.3, 9.8, 15.5, 16.4, 17.2, 17.5, 17.9, 19.9, 20.4, 20.6, 21.4, 21.7, 22.4, 22.9, 23.7, 23.9, 24.3, 25.0, 25.6, 26.4, 26.9, 27.6, 29.7 and 31.3 degrees 2θ.
7. The crystalline form of any one of the preceding paragraphs, wherein the crystalline form has a chemical purity of greater than about 90%.
8. The crystalline form of paragraph 7, wherein the chemical purity of the crystalline from is determined by HPLC analysis.
9. The crystalline form of any one of the preceding paragraphs, wherein the crystalline from has an enantiomeric purity of greater than about 90%.
10. The crystalline form of any one of the preceding paragraphs, wherein the crystalline from has a diastereomeric purity of greater than about 90%.
11. The crystalline form of any one of the preceding paragraphs, wherein the crystalline form has a melting point in the range of from about 100° C to about 140° C.
12. The crystalline form of any one of the preceding paragraphs, wherein the crystalline form has a melting point in the range of from about 110 °C to about 130 °C.
13. The crystalline form of any one of the preceding paragraphs, wherein the crystalline form has a melting point in the range of from about from about 118 °C to about 121 °C.
14. The crystalline form of any one of the preceding paragraphs, wherein the crystalline form has a melting point in the range of from about from about 119 °C to about 121 °C.
15. The crystalline form of any one of the preceding paragraphs, wherein the composition comprises at least two crystalline forms of the compound of Formula I.
16. A pharmaceutical composition comprising the crystalline form of any one of the preceding paragraphs.
17. The pharmaceutical composition of paragraph 16, wherein the pharmaceutical composition is in a solid dosage form.
18. The pharmaceutical composition of paragraph 16 or 17, wherein the pharmaceutical composition is a capsule.
19. The pharmaceutical composition of paragraph 16, wherein the pharmaceutical composition is a suspension.
20. The pharmaceutical composition of paragraph 19, wherein the pharmaceutical composition is an aqueous suspension.
21. The pharmaceutical composition of paragraph 16, wherein the pharmaceutical composition is a liquid.
22. The pharmaceutical composition of paragraph 21, wherein the pharmaceutical composition comprises a solution of the crystalline form in water.
23. The pharmaceutical composition of paragraph 21, wherein the pharmaceutical composition comprises a solution of the crystalline form in a saline solution, an aqueous dextrose solution, a glycerol solution, or a combination thereof.
24. The pharmaceutical composition of paragraph 22 or 23, wherein the pharmaceutical composition is for intravenous administration.
25. The pharmaceutical composition of any one of paragraphs 16 to 23, wherein the pharmaceutical composition is for oral administration.
26. The pharmaceutical composition of any one of paragraphs 16 to 25, further comprising one or more excipients selected from the group consisting of wetting agents, emulsifying agents, buffering agents, stabilizing agents, thickening agents, lubricating agents, and coloring agents.
27. The pharmaceutical composition of any one of paragraphs 16 to 26, wherein the pharmaceutical composition comprises at least two crystalline forms of the compound of Formula I.
28. A kit for preventing or treating a disease in a subject, the kit comprising the crystalline form of any one of paragraphs 1 to 15, or the pharmaceutical composition of any one of paragraphs 16 to 27, and instructions for using the kit.
29. The kit of paragraph 28, wherein the subject is an animal.
30. The kit of paragraph 28 or 29, wherein the subject is a human.
31. The kit of any one of paragraphs 28 to 30, wherein the disease is a pain or an inflammatory disease.
32. The kit of paragraph 31, wherein the pain is a neuropathic pain or a musculoskeletal pain.
33. The kit of paragraph 31, wherein the inflammatory disease is arthritis.
34. The kit of any one of paragraphs 28 to 33, wherein the kit further comprises at least one additional therapeutic agent.
35. The kit of paragraph 34, wherein the at least one additional therapeutic agent is an agent for treatment of a pain or a neurological disease.
36. The kit of paragraph 34 or 35, wherein the crystalline form or the pharmaceutical composition and the at least one additional therapeutic agent act additively.
37. The kit of paragraph 34 or 35, wherein the crystalline form or the pharmaceutical composition and the at least one additional therapeutic agent act synergistically.
38. A method of treating or preventing a disease in a subject in need thereof, wherein the method comprises administering to the subject the pharmaceutical composition of any one of paragraphs 16 to 27.
39. The method of paragraph 38, wherein the subject is a human.
40. The method of paragraph 38 or 39, wherein the disease is a pain or an inflammatory disease.
41. The method of paragraph 40, wherein the pain is a neuropathic pain or a musculoskeletal pain.
42. The method of paragraph 40, wherein the inflammatory disease is arthritis.
43. The method of any one of paragraphs 38 to 42, wherein the method further comprises administering at least one additional therapeutic agent to the subject.
44. The method of paragraph 43, wherein the at least one additional therapeutic agent is an agent for treatment of a pain or a neurological disease.
45. The method of paragraph 43 or 44, wherein the pharmaceutical composition and the at least one additional therapeutic agent act additively.
46. The method of paragraph 43 or 44, wherein the pharmaceutical composition and the at least one additional therapeutic agent act synergistically.
47. The method of any one of paragraph 43 to 46, wherein the pharmaceutical composition and the at least one additional therapeutic agent are administered concurrently.
48. A method of making a crystalline form of a compound of Formula I: 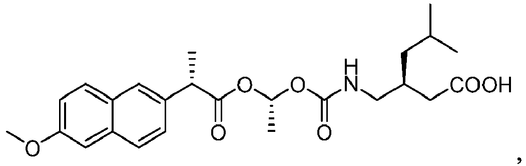 wherein the method comprises:
   (iii) dissolving a compound of Formula I in a solvent to obtain a solution of the compound of Formula I; and
   (iv) isolating the crystalline form from the solution of the compound of Formula I.
49. The method of paragraph 48, wherein the crystalline form is characterized by having XRPD peaks at about 8.6, 15.5, 16.4, 17.9, and 20.6 degrees 2θ.
50. The method of paragraph 49, wherein the crystalline form is characterized by having at least one additional XRPD peak at about 6.4, 17.2, 17.5, 20.4, 21.4, 22.4, 23.7, 23.9, or 27.6 degrees 2θ.
51. The method of paragraph 48, wherein the crystalline form is characterized by having XRPD peaks at about 6.4, 8.6, 15.5, 16.4, 17.2, 17.5, 17.9, 20.4, 20.6, 21.4, 22.4, 23.7, 23.9, and 27.6 degrees 2θ.
52. The method of any one of paragraphs 49 to 51, wherein the crystalline form is characterized by having at least one additional XRPD peak at about 9.3, 9.8, 19.9, 21.7, 22.9, 24.3, 25.0, 25.6, 26.4, 26.9, 29.7 or 31.3 degrees 2θ.
53. The method of any one of paragraphs 48 to 52, wherein the step of dissolving comprises heating the compound of Formula I and the solvent to a temperature above an ambient temperature.
54. The method of any one of paragraphs 48 to 53, wherein the step of isolating the crystalline form from the solution of the compound of Formula I comprises cooling the solution of the compound of Formula I to a temperature below an ambient temperature.
55. The method of any one of paragraphs 48 to 54, wherein the step of isolating the crystalline form from the solution of the compound of Formula I comprises cooling the solution of the compound of Formula I to a temperature of between about 0 °C and 25 °C.
56. The method of any one of paragraphs 48 to 55, wherein the method further comprises adding an additional solvent to the solution of the compound of Formula I.
57. The method of any one of paragraph 48 to 56, wherein the solvent comprises a polar protic solvent.
58. The method of any one of paragraph 48 to 57, wherein the solvent comprises isopropanol.
59. The method of any one of paragraphs 48 to 58, wherein the step of dissolving comprises heating the compound of Formula I and the solvent to a temperature of about 40 °C to about reflux temperature.
60. The method of any one of paragraphs 57 to 59, wherein the additional solvent comprises an alkane.
61. The method of any one of paragraphs 57 to 60, wherein the additional solvent comprises heptane.
62. The method of any one of the paragraphs 56 to 61, wherein the solvent and the additional solvent are used in a volume ratio of about 1:2 to about 1:3 (v/v).
63. The method of any one of paragraph 48 to 56, wherein the solvent comprises heptane, ethyl acetate, or a mixture thereof.
64. The method of paragraph 63, wherein the solvent comprises heptane and ethyl acetate in ratio of about 10:1 by volume.
65. The method of paragraph 63 or 64, wherein the step of dissolving comprises heating the compound of Formula I and the solvent to a temperature of about 50 °C to about reflux temperature.
66. The method of any one of paragraphs 63 to 65, wherein the step of dissolving comprises heating the compound of Formula I and the solvent to a temperature of about 70 °C.
67. The method of any one of paragraph 48 to 56, wherein the solvent comprises methylcyclohexane, methyl t-butyl ether, or a mixture thereof.
68. The method of paragraph 67, wherein the solvent comprises methylcyclohexane and methyl t-butyl ether in ratio of about 10:1 by volume.
69. The method of paragraph 67 or 68, wherein the step of dissolving comprises heating the compound of Formula I and the solvent to a temperature of about 20 °C to about 40 °C.
70. The method of any one of paragraphs 48 to 69, further comprising introducing a seed crystalline form into the solution of the compound of Formula I.

## Claims

1. A process for preparing (S)-3-(((((R)-1-(((S)-2-(6-methoxynaphthalen-2-yl)propanoyl)oxy)ethoxy)carbonyl)amino)methyl)-5-methylhexanoic acid (Formula I): 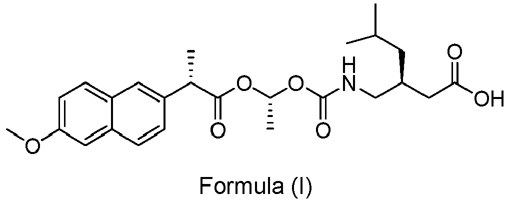
wherein the process comprises the step of reacting (S)-((R,S)-1-((2-fluorophenoxy)carbonyloxy)ethyl 2-(6-methoxynaphthalen-2-yl)propanoate (compound 4): 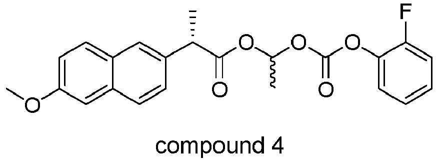
with pregabalin:

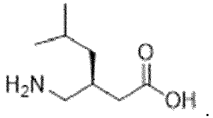

2. The process of claim 1, wherein the step of reacting compound 4 with pregabalin comprises the addition of a base.

3. The process of claim 2, wherein the base is triethylamine.

4. The process of claim 2 or 3, wherein the temperature is maintained at 15 ± 5 °C during addition of the base.

5. The process of claim 4, wherein the temperature is raised to 25 ± 3 °C after addition of the base.

6. The process of any one of claims 1-5, wherein the process comprises the step of reacting naproxen: 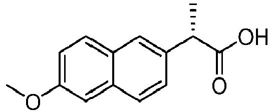
with 1-chloroethyl (2-fluorophenyl) carbonate (compound 3): 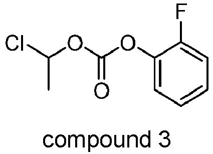
to form (S)-((R,S)-1-((2-fluorophenoxy)carbonyloxy)ethyl 2-(6-methoxynaphthalen-2-yl)propanoate (compound 4):

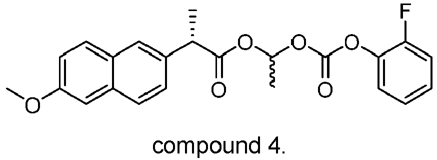

7. The process of claim 6, wherein the step of reacting naproxen with compound 3 further comprises cuprous oxide (Cu₂O).

8. The process of claim 6 or 7, wherein the step of reacting naproxen with compound 3 further comprises dissolving the naproxen in toluene.

9. The process of any one of claims 6-8, wherein the temperature of the reaction mixture in the step of reacting naproxen with compound 3 is raised to 115 ± 5 °C.

10. The process of any one of claims 1-9, wherein the process further comprises the step of recrystallizing the compound of Formula I.

11. The process of claim 10, wherein the recrystallizing comprises dissolving the compound of Formula I in a solution of isopropanol and water.

12. A crystalline form of (S)-3-(((((R)-1-(((S)-2-(6-methoxynaphthalen-2-yl)propanoyl)oxy)ethoxy)carbonyl)amino)methyl)-5-methylhexanoic acid (Formula I): 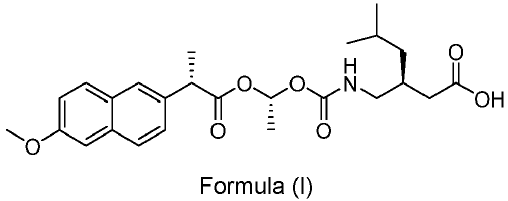
made by the process of claim 6 or 7;
wherein the crystalline form is **characterized by** having X-ray powder diffraction (XRPD) peaks at about 8.6, 15.5, 16.4, 17.9, and 20.6 degrees 2θ.

13. The crystalline form of claim 12, wherein the crystalline form is **characterized by** having at least one additional XRPD peak at about 6.4, 17.2, 17.5, 20.4, 21.4, 22.4, 23.7, 23.9, or 27.6 degrees 2θ.

14. A compound that is (S)-((R,S)-1-((2-fluorophenoxy)carbonyloxy)ethyl 2-(6-methoxynaphthalen-2-yl)propanoate (compound 4):

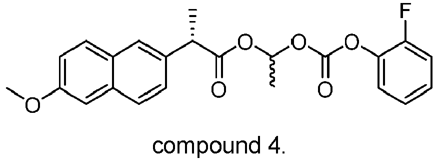

15. A compound that is (R)-1-(((2-fluorophenoxy)carbonyl)oxy)ethyl (S)-2-(6-methoxynaphthalen-2-yl)propanoate:
